# EUROPEAN PATENT APPLICATION

(11) **EP 4 834 335 A1**
(43) Date of publication of application: **04.11.2026**
(21) Application number: 24913676.3
(22) Date of filing: 27.12.2024
(51) Int. Cl.: A61K 9/51, A61K 31/7105, A61K 48/00

(54) **NANOPARTICLE COMPOSITION FOR DRUG DELIVERY**

(30) Priority: 29.12.2023 KR 20230196984
(71) Applicant: SAMYANG BIOPHARM CORPORATION, Seongnam-si Gyeonggi-do 13488 (KR)
(72) Inventor: CHOI, Ji Hye, Seoul 05615 (KR); NAM, Joung Pyo, Seoul 02471 (KR); SEO, Won Il, Seongnam-si Gyeonggi-do 13488 (KR)
(74) Representative: Gleiss Große Schrell und Partner mbB
(86) International application number: PCT/KR2024/021277
(87) International publication number: WO 2025/143871

(57) **Abstract**

The present invention relates to a composition for drug delivery and a preparation method thereof and, more specifically, to: a composition for drug delivery in which a drug is encapsulated inside a nanoparticle structure formed by a specific polymer and a cationic compound; and a preparation method thereof.

## Description

### TECHNICAL FIELD

The present invention relates to a composition for drug delivery and a method for preparing the same, and more specifically, a composition for drug delivery which is in a form where a drug is encapsulated within a nanoparticle structure formed by a specific polymer and a cationic compound, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. Thus, recent researches proceed in the direction to improve such disadvantages by using non-viral carriers. Although non-viral carriers are less efficient in comparison with viral carriers, they have advantages of fewer side effects in terms of *in vivo* safety.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted. However, when they are administered intravenously in an amount required to obtain a sufficient effect, severe toxicity is caused, although less toxic than viral carriers, resulting in that they are unsuitable for use in pharmaceutical products. Accordingly, there is a need to develop anionic drug delivery technology that is stable *in vivo* and capable of intracellular delivery to obtain sufficient effects, while reducing toxicity by minimizing the use of cationic polymer or cationic lipid that may cause toxicity.

Various anionic drug delivery compositions and preparation methods thereof have been disclosed, wherein a complex is formed by electrostatic interaction between nucleic acid and cationic lipid, and said complex is encapsulated within a nanoparticle structure of amphiphilic block copolymer. For example, Korean Laid-open Patent Publication No. 10-2017-0032858 discloses a composition for delivering an anionic drug, comprising the anionic drug as an active ingredient; a cationic compound; an amphiphilic block copolymer; and a salt of polylactic acid, wherein the anionic drug forms a complex with the cationic compound by electrostatic interaction, and the formed complex is encapsulated within the nanoparticle structure formed by the amphiphilic block copolymer and the salt of polylactic acid, and a method for preparing the same. However, including those disclosed in the above patent publication, the existing nanoparticle drug delivery systems still lack the efficiency to deliver drugs such as nucleic acid, polypeptide, or virus (especially mRNA) into the body.

### CONTENTS OF THE INVENTION

### PROBLEMS TO BE SOLVED

The purpose of the present invention is to provide a composition for drug delivery having significantly improved *in vivo* delivery efficiency of drugs such as nucleic acid, polypeptide, or virus (especially mRNA), as compared with previously known nanoparticle drug delivery systems, and a method for preparing the same.

### TECHNICAL MEANS

The first aspect of the present invention provides a composition for drug delivery comprising: effective ingredient selected from nucleic acid, polypeptide, virus or combination thereof; an amphiphilic block copolymer comprising a hydrophilic block and a hydrophobic block; and a cationic compound, wherein the hydrophobic block is a biocompatible, biodegradable polymer having a repeating unit represented by the following Formula 1: 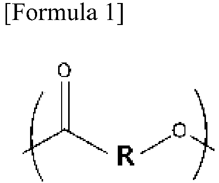
wherein, in the above Formula 1,
R represents a branched alkylene group having 3 or more carbon atoms.

The second aspect of the present invention provides a method for preparing a composition for drug delivery, comprising the steps of: (a) preparing a solution in which an amphiphilic block copolymer comprising a hydrophilic block and a hydrophobic block; and a cationic compound; are dissolved in a water-miscible organic solvent; and (b) to the solution prepared in step (a), adding effective ingredient selected from nucleic acid, polypeptide, virus, or combination thereof, and mixing them, wherein the hydrophobic block is a biocompatible, biodegradable polymer having a repeating unit represented by the above Formula 1.

### EFFECT OF THE INVENTION

The composition for drug delivery according to the present invention can significantly improve *in vivo* delivery efficiency of drugs such as nucleic acid, polypeptide, or virus (especially mRNA), as compared with previously known nanoparticle drug delivery systems.

### CONCRETE MODE FOR CARRYING OUT THE INVENTION

The present invention will be explained in detail below.

### Effective ingredient

The effective ingredient comprised in the composition for drug delivery of the present invention is selected from nucleic acid, polypeptide, virus, or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be an oncolytic virus and, for example, may be one or more selected from the group consisting of adenovirus, AAV, vaccinia virus, herpes simplex virus (HSV), and vesicular stomatitis virus (VSV). In an embodiment, the oncolytic virus is an adenovirus. The adenovirus used in an embodiment of the present invention contains a luciferase gene, which can be confirmed through imaging.

The virus can express various types of therapeutic genes within the body of an individual and is not limited to specific molecular weight, protein, bioactivity or therapeutic field. The prophylactic virus can induce immunity within the body of an individual against a target disease. A composition containing a prophylactic virus to disease has the advantage of reducing immunity induction by the virus itself, capability of designating or expanding target cells, and reducing the hyperimmune response to the virus upon re-administration, thereby enabling effective effects to be obtained through multiple inoculations.

In an embodiment, the effective ingredient is mRNA (messenger RNA).

The mRNA may be chemically modified in its backbone, sugar, or base, or may be terminally modified, for purposes such as increasing stability in the bloodstream or reducing an immune response.

In an embodiment, the amount of the effective ingredient may be, based on the dry weight of the total composition, 0.05 wt% or more, 0.1 wt% or more, 0.2 wt% or more, 0.3 wt% or more, 0.4 wt% or more, or 0.5 wt% or more, and it may also be 10 wt% or less, 9 wt% or less, 8 wt% or less, 7 wt% or less, 6 wt% or less, 5 wt% or less, 4 wt% or less, or 3 wt% or less. If the amount of the effective ingredient is too less, the amount of delivery carrier becomes too much as compared with the drug, and thus there may be a side effect due to the delivery carrier. To the contrary, if the amount of the effective ingredient is too much, the amount of drug not encapsulated in nanoparticles becomes too much, and thus the efficiency decreases.

### Amphiphilic block copolymer

The composition for drug delivery of the present invention comprises an amphiphilic block copolymer comprising a hydrophilic block and a hydrophobic block, wherein the hydrophobic block is a biocompatible, biodegradable polymer having a repeating unit represented by the following Formula 1: 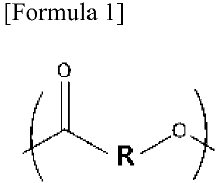
wherein, in the above Formula 1,
R represents a branched alkylene group having 3 or more carbon atoms.

In an embodiment, the number of repeating units (degree of polymerization) of the hydrophobic block may be, for example, 1 or more, 2 or more, 3 or more, 4 or more, 5 or more, 6 or more, 7 or more, 8 or more, 9 or more, or 10 or more, and may also be 25 or less, 24 or less, 23 or less, 22 or less, 21 or less, or 20 or less, but is not limited thereto.

In an embodiment, the amphiphilic block copolymer may be an A-B type block copolymer comprising a hydrophilic block (A) and a hydrophobic block (B). In an aqueous environment, the A-B type block copolymer forms core-shell type polymer nanoparticle wherein the hydrophobic block (B) forms the core (inner wall) and the hydrophilic block (A) forms the shell (outer wall).

In an embodiment, the hydrophilic block may be one or more selected from the group consisting of polyalkylene glycol, polyvinyl alcohol, polyvinyl pyrrolidone, polyacrylamide, and derivatives thereof.

More specifically, the hydrophilic block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

In an embodiment, the number average molecular weight (g/mol) of the hydrophilic block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand includes any molecule that is capable of directly or indirectly interacting with another compound such as a receptor, including, but not limited to, an amino acid, sugar, vitamin, peptide, protein, hormone, antibody, neurotransmitter, pharmaceutically active small molecule, endosome-disrupting agent, cell membrane-permeabilizing agent, charge-masking agent, drug, nucleic acid, or a derivative thereof. The sugar may include, but is not limited to, galactose, galactosamine, N-acetylgalactosamine, or a combination thereof. The hormone may include, but is not limited to, estrogen, testosterone, progesterone, glucocortisone, adrenaline, insulin, glucagon, cortisol, vitamin D, thyroid hormone, retinoic acid, growth hormone, or a combination thereof. The neurotransmitter may include, but is not limited to, growth factors such as VEGF, EGF, NGF, and PDGF; cholesterol; bile acids; GABA; glutamate; acetylcholine; or a combination thereof. In an embodiment, the functional group or ligand may be attached to the terminus of the hydrophilic block through a linker molecule. The linker molecule may include, but is not limited to, an amide, carbonyl, ester, peptide, disulfide, silane, nucleoside, abasic nucleoside, polyether, polyamine, polyamide, carbohydrate, lipid, polyhydrocarbon, phosphate ester, phosphoramidate, thiophosphate, alkyl phosphate, biodegradable linker, photolabile linker, or a combination thereof.

The hydrophobic block is a biocompatible, biodegradable polymer having a repeating unit represented by the above Formula 1.

In an embodiment, the number of carbon atoms of R in the above Formula 1 may be, for example, 3 or more, 4 or more, 5 or more, 6 or more, or 7 or more, and may also be 20 or less, 19 or less, 18 or less, 17 or less, 16 or less, 15 or less, 14 or less, or 13 or less, but is not limited thereto.

In an embodiment, R in the above Formula 1 may represent a branched alkylene group having 3 to 20 carbon atoms, more specifically a branched alkylene group having 3 to 17 carbon atoms, still more specifically a branched alkylene group having 3 to 15 carbon atoms, and even more specifically a branched alkylene group having 3 to 13 carbon atoms, but is not limited thereto.

In an embodiment, the hydrophobic block may be a biocompatible, biodegradable polymer having repeating units of a structure selected from the following, but is not limited thereto:

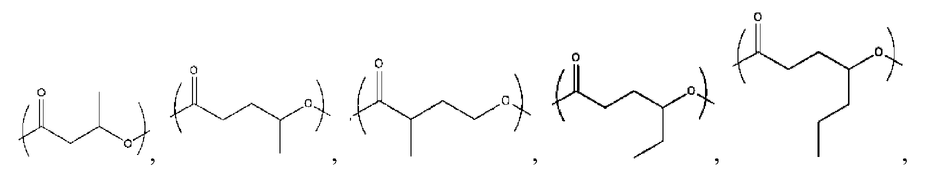

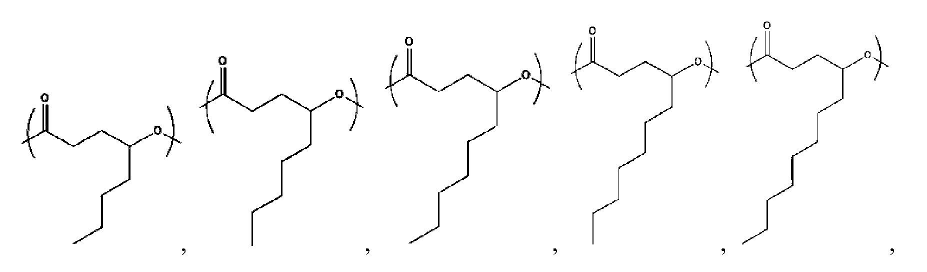

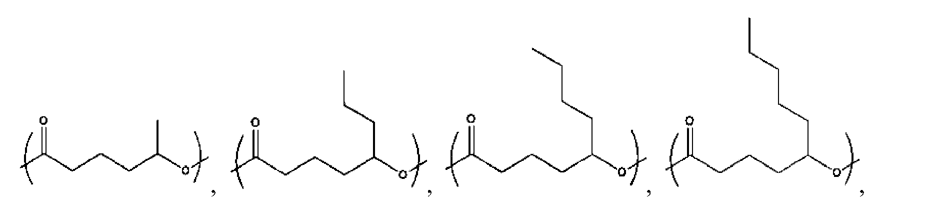

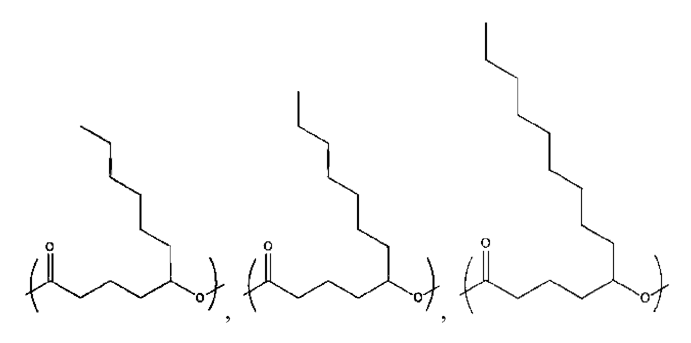

In an embodiment, the repeating unit represented by the above Formula 1 may be obtained by ring-opening polymerization of a lactone compound.

In an embodiment, the number average molecular weight (g/mol) of the hydrophobic block may be 80 or more, 100 or more, 150 or more, 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic block-hydrophobic block may be 2,000-6,000, 2,000-4,000, 2,000-3,000, 2,000-1,700, 2,000-1,300, 2,000-1,000, 2,000-800, 2,000-500, etc., but it is not limited thereto.

Also, in an embodiment, in order to increase the hydrophobicity of the hydrophobic block and thereby improve the stability of the nanoparticle, the hydrophobic block may be modified by chemically combining the hydroxyl group at the end of the hydrophobic block with tocopherol, cholesterol, or a fatty acid having 10 to 24 carbon atoms.

In an embodiment, in the amphiphilic block copolymer, the amount ratio of the hydrophilic block and the hydrophobic block may be such that the hydrophilic block is present in an amount of 25 to 95 wt%, specifically 40 to 90 wt%, and more specifically 50 to 80 wt%, based on the total weight of the copolymer. If the ratio of the hydrophilic block is less than 25 wt% based on the total weight of the copolymer, the solubility of the polymer in water is low, making it difficult to form nanoparticles. Thus, in order for the copolymer to have sufficient water solubility to form nanoparticles, it is preferable that the ratio of the hydrophilic block be 25 wt% or more. To the contrary, if the ratio of the hydrophilic block is greater than 95 wt% based on the total weight of the copolymer, the hydrophilicity becomes too high, lowering the stability of the polymer nanoparticles and making it difficult to use the copolymer as a solubilizing composition of effective ingredient-containing complex. Thus, considering the stability of the nanoparticles, it is preferable that the ratio of the hydrophilic block be 95 wt% or less.

In an embodiment, the amount of the amphiphilic block copolymer in the composition for drug delivery of the present invention may be, based on the dry weight of the total composition, 5 wt% or more, 6 wt% or more, 7 wt% or more, 10 wt% or more, 12 wt% or more, 15 wt% or more, 17 wt% or more, or 18 wt% or more, and it may also be 90 wt% or less, 80 wt% or less, 70 wt% or less, 60 wt% or less, 50 wt% or less, 40 wt% or less, or 30 wt% or less. If the amount of the polymer is too less, the size of the nanoparticle becomes too large, and thus the nanoparticle stability may be lowered and the rate of loss during filter sterilization may increase. To the contrary, if the amount of the polymer is too much, there is a concern that the amount of the effective ingredient that can be incorporated will become too less.

According to an embodiment, within a range that allows achievement of the object of the present invention, the hydrophobic block may further comprise an additional hydrophobic repeating unit other than the repeating unit having the structure represented by the above Formula 1.

In an embodiment, the additional hydrophobic repeating unit may be one or more selected from the group consisting of polyester, polyanhydride, polyamino acid, polyorthoester and polyphosphazene. More specifically, the additional hydrophobic repeating unit may be one or more selected from the group consisting of polylactide (PLA), polyglycolide, polydioxan-2-one, a copolymer of lactide and glycolide, and a copolymer of lactide and dioxan-2-one, but is not limited thereto.

### Cationic compound

The composition for drug delivery of the present invention comprises a cationic compound.

In an embodiment, the cationic compound may be a cationic lipid, a cationic polymer, or a combination thereof, and more specifically, may be a cationic lipid.

For example, the cationic lipid may be one or a combination of two or more selected from the group consisting of N,N-dioleyl-N,N-dimethylammonium chloride (DODAC), N,N-distearyl-N,N-dimethylammonium bromide (DDAB), N-(1-(2,3-dioleoyloxy)propyl)-N,N,N-trimethylammonium chloride (DOTAP), N,N-dimethyl-(2,3-dioleoyloxy)propylamine (DODMA), N,N,N-trimethyl-(2,3-dioleoyloxy)propylamine (DOTMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3β-[N-(N',N'-dimethylaminoethane)carbamoyl] cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), cholesteryloxypropan-1-amine (COPA), N-(N'-aminoethane)carbamoylpropanoic tocopherol (AC-tocopherol), and N-(N'-methylaminoethane)carbamoylpropanoic tocopherol (MC-tocopherol). More specifically, the cationic lipid may be one or more selected from the group consisting of 3β-[N-(N',N',N'-trimethylaminoethane)carbamoyl]cholesterol (TC-cholesterol), 3β-[N-(N',N'-dimethylaminoethane)carbamoyl]cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), N-(1-(2,3-dioleoyloxy)propyl)-N,N,N-trimethylammonium chloride (DOTAP), N,N-dimethyl-(2,3-dioleoyloxy)propylamine (DODMA), and N,N,N-trimethyl-(2,3-dioleoyloxy)propylamine (DOTMA).

In an embodiment, the cationic lipid may be a lipid having a structure represented by the following Formula 2, or an ionized form thereof: 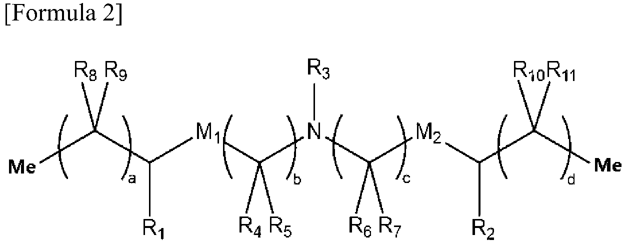
wherein, in the above Formula 2,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

In the above Formula 2, the expression "substituted or unsubstituted" for any group means that, unless specified otherwise, the group is not substituted, or is substituted with hydroxy group or C₁₋₆ alkyl group.

According to an embodiment, in the above Formula 2, each of M₁ and M₂ may be independently selected from the group consisting of -C(O)O-, -OC(O)-, -OC(O)-M'-C(O)O-, - C(O)N(R')-, -N(R')C(O)-, -C(O)-, -C(S)-, -C(S)S-, -SC(S)-, -CH(OH)-, -P(O)(OR')O-, -S(O)₂-, - S-S-, arylene (more specifically C₆₋₂₀ arylene, still more specifically C₆₋₁₀ arylene), and heteroarylene (more specifically C₃₋₂₀ heteroarylene, still more specifically C₃₋₁₀ heteroarylene, having one or more (e.g., 1 to 3) heteroatoms selected from N, O and S), wherein M' may be a direct bond, C₁₋₁₃ alkylene (more specifically C₁₋₆ alkylene) or C₂₋₁₃ alkenylene (more specifically C₂₋₆ alkenylene), and each R' may be independently selected from the group consisting of hydrogen atom, C₁₋₁₈ alkyl (more specifically C₁₋₁₀ alkyl, still more specifically C₁₋₆ alkyl) and C₂₋₁₈ alkenyl (more specifically C₂₋₁₀ alkenyl, still more specifically C₂₋₆ alkenyl).

According to an embodiment, in the above Formula 2, each of R₁ and R₂ may be independently selected from the group consisting of substituted or unsubstituted C₃₋₂₀ cycloalkyl (more specifically C₃₋₁₀ cycloalkyl, still more specifically C₃₋₆ cycloalkyl), substituted or unsubstituted C₃₋₂₀ cycloalkenyl (more specifically C₃₋₁₀ cycloalkenyl, still more specifically C₃₋₆ cycloalkenyl), substituted or unsubstituted C₆₋₂₀ aryl (more specifically C₆₋₁₀ aryl, still more specifically C₆ aryl), substituted or unsubstituted C₃₋₂₀ heterocycloalkyl (more specifically C₃₋₁₀ heterocycloalkyl, still more specifically C₃₋₆ heterocycloalkyl), substituted or unsubstituted C₃₋₂₀ heterocycloalkenyl (more specifically C₃₋₁₀ heterocycloalkenyl, still more specifically C₃₋₆ heterocycloalkenyl), and substituted or unsubstituted C₃₋₂₀ heteroaryl (more specifically C₃₋₁₀ heteroaryl, still more specifically C₃₋₆ heteroaryl), wherein each of the heterocycloalkyl, heterocycloalkenyl and heteroaryl may independently have one or more (e.g., 1 to 3) heteroatoms selected from N, O and S.

According to an embodiment, in the above Formula 2, R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, - O(CH₂)ₙN(R)₂, -C(O)OR, -OC(O)R, -CX₃, -CX₂H, -CXH₂, -CN, -N(R)₂, -C(O)N(R)₂, - N(R)C(O)R, -N(R)S(O)₂R, -N(R)C(O)N(R)₂, -N(R)C(S)N(R)₂, -N(R)R₁₂, N(R)S(O)₂R₁₂, - O(CH₂)ₙOR, -N(R)C(=NR₁₃)N(R)₂, -N(R)C(=CHR₁₃)N(R)₂, -OC(O)N(R)₂, -N(R)C(O)OR, - N(OR)C(O)R, -N(OR)S(O)₂R, -N(OR)C(O)OR, -N(OR)C(O)N(R)₂, -N(OR)C(S)N(R)₂, - N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, -S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is -(CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment, in the above Formula 2, each of R₄ to R₁₁ may be independently selected from the group consisting of hydrogen atom, C₁₋₃ alkyl and C₂₋₃ alkenyl.

According to an embodiment, in the above Formula 2, each of a, b, c and d may be independently an integer of from 1 to 15.

Still more specifically, in the above Formula 2, each of M₁ and M₂ may be independently -C(O)O- or -OC(O)-.

Still more specifically, in the above Formula 2, each of R₁ and R₂ may be independently substituted or unsubstituted C₃₋₆ cycloalkyl.

Still more specifically, in the above Formula 2, R₃ may be hydrogen atom, or substituted or unsubstituted C₁₋₃ alkyl, and even more specifically, unsubstituted C₁₋₃ alkyl or hydroxy-substituted C₁₋₃ alkyl.

Still more specifically, in the above Formula 2, R₄ to R₁₁ may be hydrogen atom.

Still more specifically, in the above Formula 2, each of a, b, c and d may be independently an integer of from 3 to 11, and even more specifically, an integer of from 5 to 9.

Even more specifically, the lipid of the above Formula 2 may be one having a structure selected from the following Formulas 2-A to 2-O:

| Formula | Structure |
|---|---|
| 2-A | 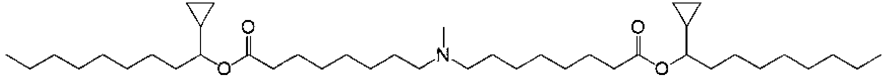 |
| 2-B | 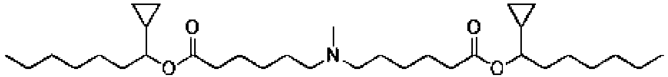 |
| 2-C | 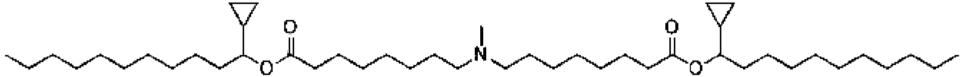 |
| 2-D | 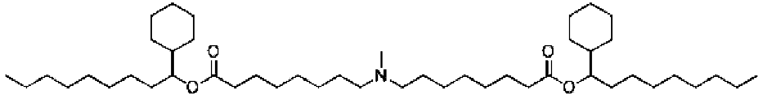 |
| 2-E | 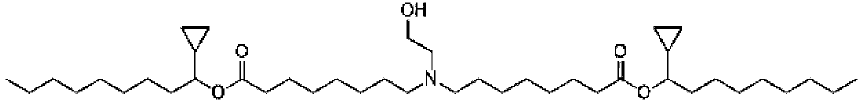 |
| 2-F | 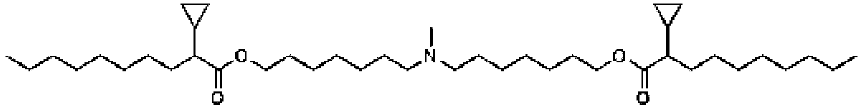 |
| 2-G | 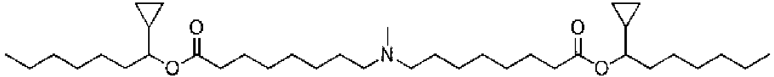 |
| 2-H | 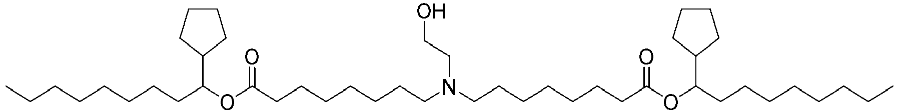 |
| 2-I | 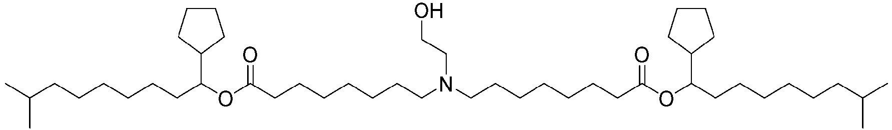 |
| 2-J | 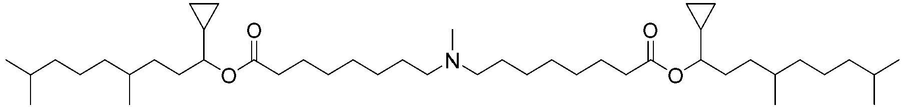 |
| 2-K | 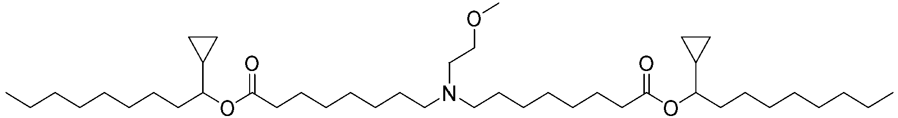 |
| 2-L | 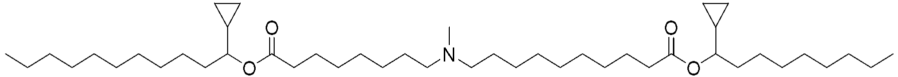 |
| 2-M | 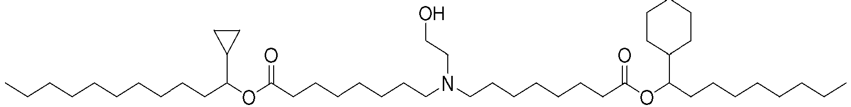 |
| 2-N | 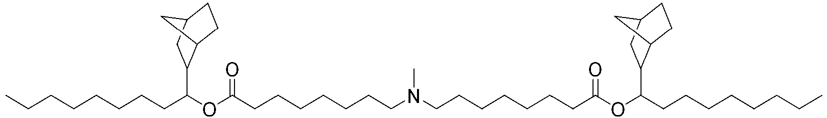 |
| 2-O | 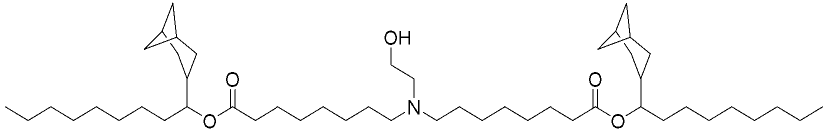 |

In other embodiment, the cationic lipid may be a lipid having a structure among those shown in the following Formula 3, or an ionized form thereof: 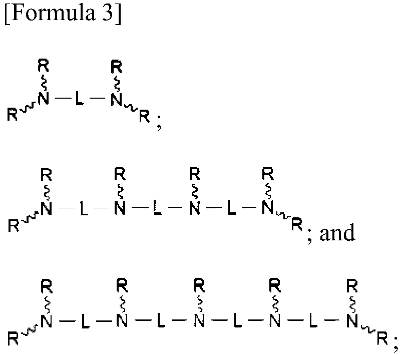
wherein, in each of the structures shown in the above Formula 3,
at least two (more specifically, 2 to 7) of R groups are Rx, and other R groups are Ry, wherein
each Rx is independently selected from 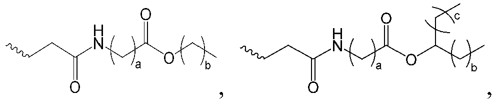 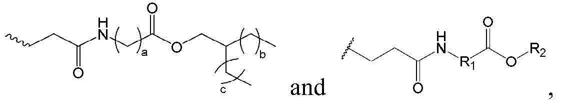 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 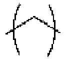 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

In the above Formula 3, the expression "substituted or unsubstituted" for any group means that, unless specified otherwise, the group is not substituted, or is substituted with one or more substituents selected from -OH, halogen atom, C₁₋₆ alkyl group, C₁₋₆ alkoxy group, C₁₋₆ halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

According to an embodiment, in the above Formula 3, each Rx is independently selected from 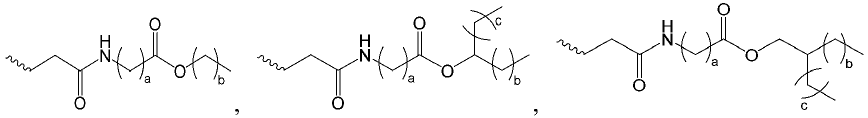 and 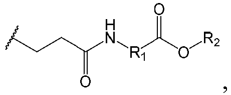 where each of a, b and c may be independently an integer of from 2 to 20 or from 2 to 15, R₁ may be substituted or unsubstituted, saturated or unsaturated divalent C₁₋₁₂ hydrocarbon group, R₂ may be substituted or unsubstituted, unsaturated monovalent C₂₋₂₄ hydrocarbon group, and 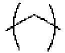 represents substituted or unsubstituted methylene group.

According to an embodiment, in the above Formula 3, each of a, b and c may be independently an integer of from 2 to 15, and more specifically, may be independently an integer of from 3 to 12. Even more specifically, a may be independently an integer of from 5 to 7, and each of b and c may be independently an integer of from 3 to 11, but it is not limited thereto.

According to an embodiment, in the above Formula 3, each Ry may be independently H or C₁₋₂₀ alkyl group, where the alkyl group may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O. Also, in the above, the alkyl or alkoxy group may be more specifically C₁₋₁₀ alkyl or alkoxy group, and still more specifically C₁₋₆ alkyl or alkoxy group, but it is not limited thereto.

According to an embodiment, in the above Formula 3, each L may be independently C₁₋₂₀ alkylene group (more specifically C₁₋₁₀ alkylene group, and still more specifically C₁₋₆ alkylene group), each of which may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S. Also, in the above, the alkyl or alkoxy group may be more specifically C₁₋₁₀ alkyl or alkoxy group, and still more specifically C₁₋₆ alkyl or alkoxy group, but it is not limited thereto.

More specifically, in the above Formula 3, each Ry may be independently H or C₁₋₁₀ alkyl group, where the alkyl group may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

More specifically, in the above Formula 3, each L may be independently C₁₋₁₀ alkylene group, each of which may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S.

Even more specifically, in the above Formula 3, each Rx is independently selected from 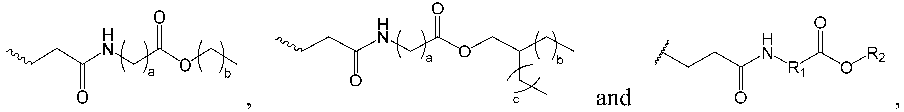 where each of a, b and c may be independently an integer of from 3 to 12, R₁ may be substituted or unsubstituted C₁₋₁₂ alkylene group, substituted or unsubstituted C₂₋₁₂ alkenylene group or substituted or unsubstituted C₂₋₁₂ alkynylene group, R₂ may be substituted or unsubstituted C₂₋₂₄ alkenyl group or substituted or unsubstituted C₂₋₂₄ alkynyl group, and 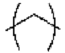 represents substituted or unsubstituted methylene group.

Even more specifically, in the above Formula 3, each Ry may be independently H or C₁₋₆ alkyl group, where the alkyl group may be independently unsubstituted, or may be substituted with one or more selected from -OH and -NH₂; and two Ry groups that are not H may be connected together with nitrogen atom to which they are attached, to form a ring structure optionally having one or more heteroatoms selected from N and O.

Even more specifically, in the above Formula 3, each L may be independently unsubstituted C₁₋₆ alkylene group.

Specifically, the lipid of the above Formula 3 may have a structure selected from the following:

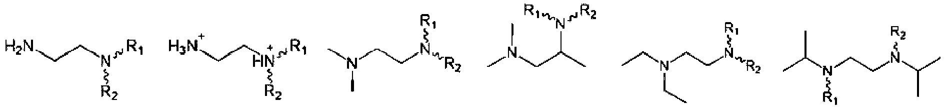

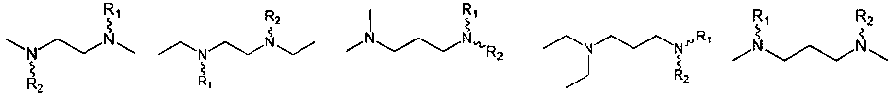

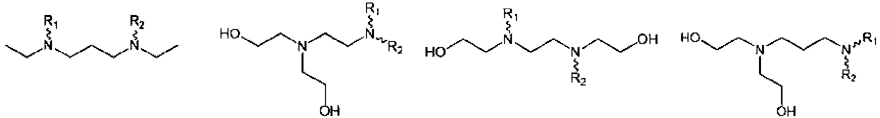

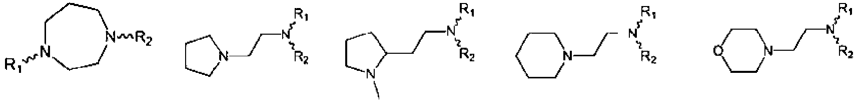

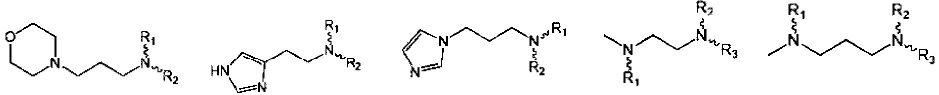

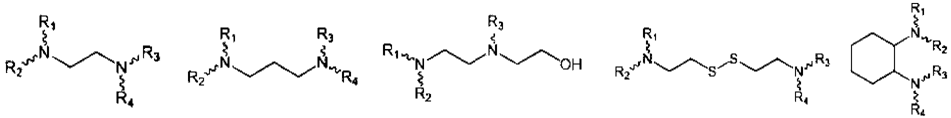

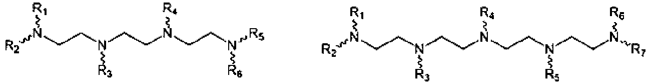

In each of the above structures, each of R₁ to R₇ is independently selected from 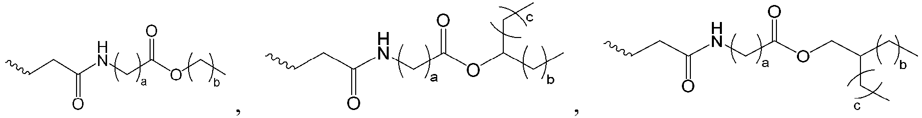 and 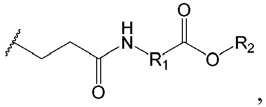 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 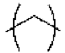 represents substituted or unsubstituted methylene group.

Still more specifically, the lipid of the above Formula 3 may be one having a structure selected from the following Formulas 3-A to 3-V:

| Formula | Structure |
|---|---|
| 3-A | 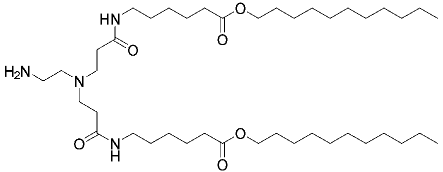 |
| 3-B | 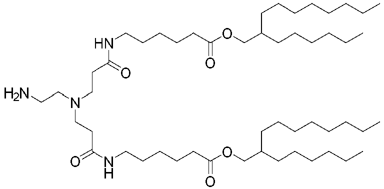 |
| 3-C | 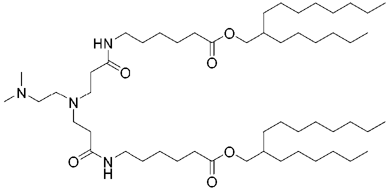 |
| 3-D | 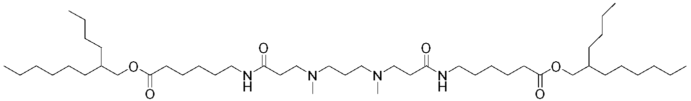 |
| 3-E | 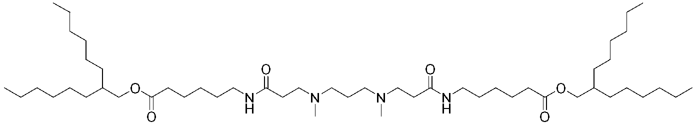 |
| 3-F | 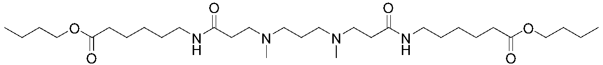 |
| 3-G | 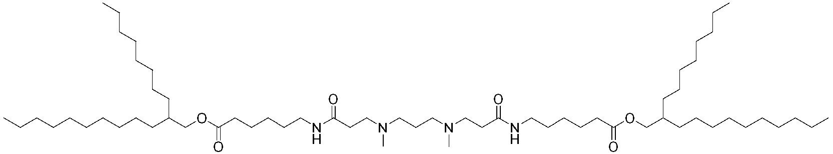 |
| 3-H | 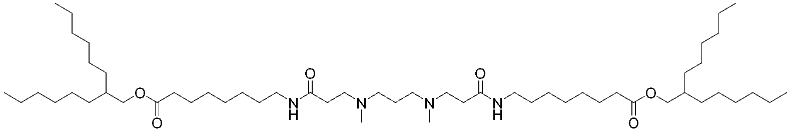 |
| 3-I | 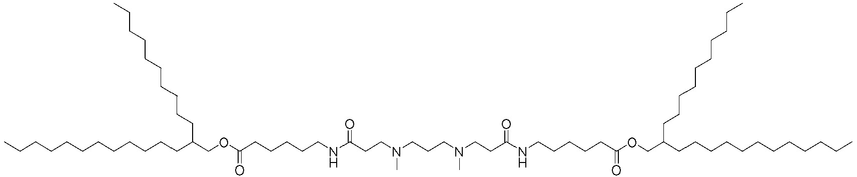 |
| 3-J | 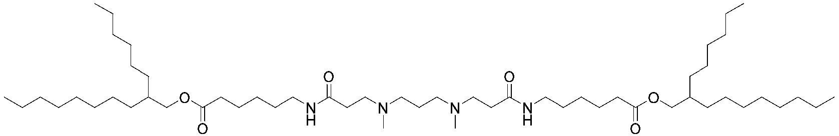 |
| 3-K | 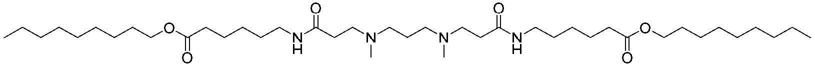 |
| 3-L | 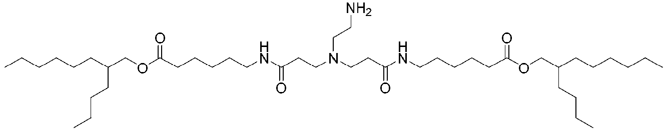 |
| 3-M | 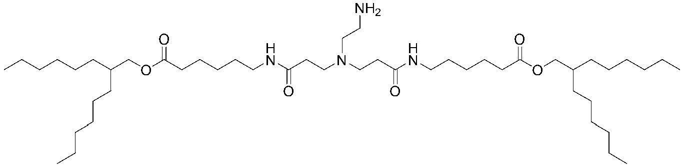 |
| 3-N | 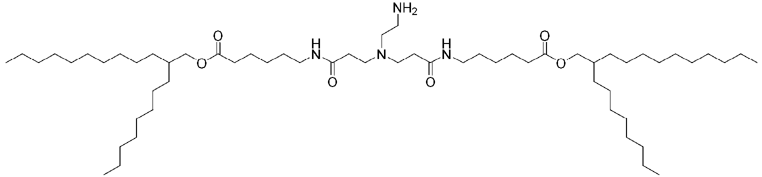 |
| 3-O | 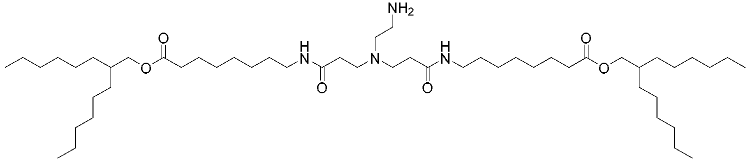 |
| 3-P | 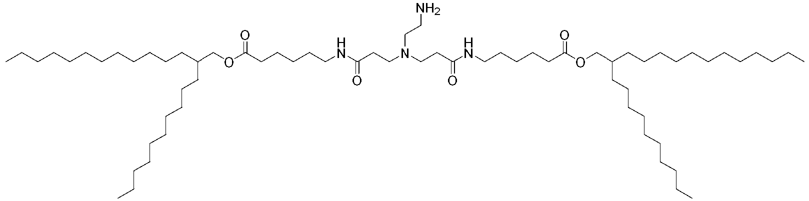 |
| 3-Q | 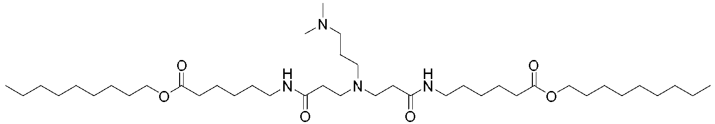 |
| 3-R | 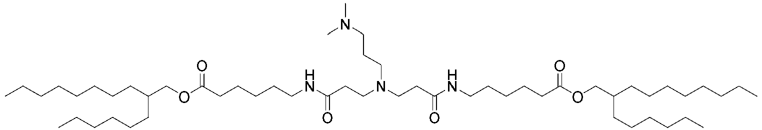 |
| 3-S | 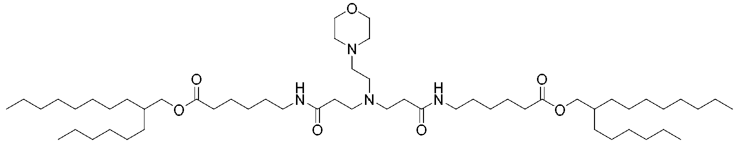 |
| 3-T | 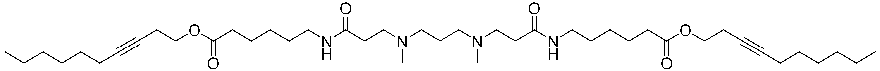 |
| 3-U | 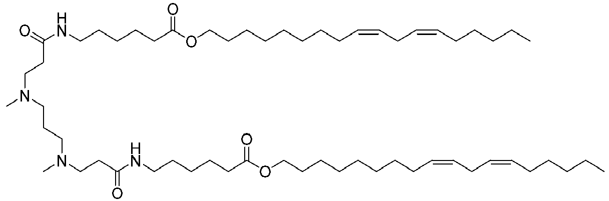 |
| 3-V | 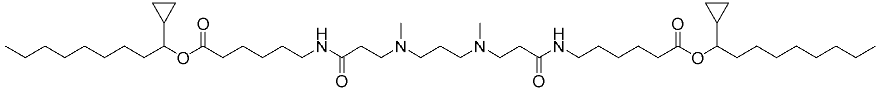 |

In another embodiment, the cationic lipid may be a lipid having a structure represented by the following Formula 4, or an ionized form thereof: 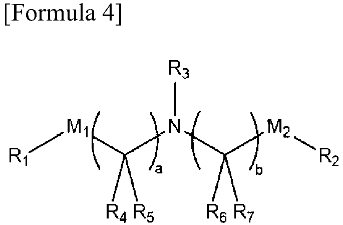
wherein, in the above Formula 4,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

In the above Formula 4, the expression "substituted or unsubstituted" for any group means that, unless specified otherwise, the group is not substituted, or is substituted with one or more substituents selected from -OH, halogen atom, C₁₋₈ alkyl group (more specifically, C₃₋₇ alkyl group) or C₁₋₈ halogenated alkyl group (more specifically, C₃₋₇ halogenated alkyl group).

According to an embodiment, in the above Formula 4, each of M₁ and M₂ may be independently selected from the group consisting of -C(O)O-, -OC(O)-, -OC(O)-M'-C(O)O-, - C(O)N(R')-, -N(R')C(O)-, -C(O)-, -C(S)-, -C(S)S-, -SC(S)-, -CH(OH)-, -P(O)(OR')O-, -S(O)₂-, - S-S-, arylene (more specifically C₆₋₂₀ arylene, still more specifically C₆₋₁₀ arylene), and heteroarylene (more specifically C₃₋₂₀ heteroarylene, still more specifically C₃₋₁₀ heteroarylene, having one or more (e.g., 1 to 3) heteroatoms selected from N, O and S), wherein M' may be a direct bond, C₁₋₁₃ alkylene (more specifically C₁₋₆ alkylene) or C₂₋₁₃ alkenylene (more specifically C₂₋₆ alkenylene), and each R' may be independently selected from the group consisting of hydrogen atom, C₁₋₁₈ alkyl (more specifically C₁₋₁₀ alkyl, still more specifically C₁₋₆ alkyl) and C₂₋₁₈ alkenyl (more specifically C₂₋₁₀ alkenyl, still more specifically C₂₋₆ alkenyl).

According to an embodiment, in the above Formula 4, each of R₁ and R₂ may be independently selected from the group consisting of substituted or unsubstituted C₃₋₂₀ cycloalkyl (more specifically C₃₋₁₅ cycloalkyl, still more specifically C₆₋₁₅ cycloalkyl), substituted or unsubstituted C₃₋₂₀ cycloalkenyl (more specifically C₃₋₁₅ cycloalkenyl, still more specifically C₆₋₁₅ cycloalkenyl), substituted or unsubstituted C₆₋₂₀ aryl (more specifically C₆₋₁₄ aryl), substituted or unsubstituted C₃₋₂₀ heterocycloalkyl (more specifically C₃₋₁₅ heterocycloalkyl), substituted or unsubstituted C₃₋₂₀ heterocycloalkenyl (more specifically C₃₋₁₅ heterocycloalkenyl), and substituted or unsubstituted C₃₋₂₀ heteroaryl (more specifically C₃₋₁₅ heteroaryl), wherein each of the heterocycloalkyl, heterocycloalkenyl and heteroaryl may independently have one or more (e.g., 1 to 3) heteroatoms selected from N, O and S.

According to an embodiment, in the above Formula 4, R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, - O(CH₂)ₙN(R)₂, -C(O)OR, -OC(O)R, -CX₃, -CX₂H, -CXH₂, -CN, -N(R)₂, -C(O)N(R)₂, - N(R)C(O)R, -N(R)S(O)₂R, -N(R)C(O)N(R)₂, -N(R)C(S)N(R)₂, -N(R)R₁₂, N(R)S(O)₂R₁₂, - O(CH₂)ₙOR, -N(R)C(=NR₁₃)N(R)₂, -N(R)C(=CHR₁₃)N(R)₂, -OC(O)N(R)₂, -N(R)C(O)OR, - N(OR)C(O)R, -N(OR)S(O)₂R, -N(OR)C(O)OR, -N(OR)C(O)N(R)₂, -N(OR)C(S)N(R)₂, - N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, -S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is -(CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment, in the above Formula 4, each of R₄ to R₇ may be independently selected from the group consisting of hydrogen atom, C₁₋₃ alkyl and C₂₋₃ alkenyl.

According to an embodiment, in the above Formula 4, each of a and b may be independently an integer of from 1 to 15, and even more concretely, an integer of from 3 to 13.

Still more specifically, in the above Formula 4, each of M₁ and M₂ may be independently selected from the group consisting of -C(O)O-, -OC(O)-, -C(O)N(R')- and -N(R')C(O)-, wherein R' is the same as defined above.

Still more specifically, in the above Formula 4, each of R₁ and R₂ may be independently substituted or unsubstituted C₃₋₁₅ cycloalkyl.

Still more specifically, in the above Formula 4, R₃ may be hydrogen atom, or substituted or unsubstituted C₁₋₃ alkyl.

Still more specifically, in the above Formula 4, R₄ to R₇ may be hydrogen atom.

Still more specifically, in the above Formula 4, each of a and b may be independently an integer of from 5 to 11, and even more specifically, an integer of from 5 to 9.

Even more specifically, the lipid of the above Formula 4 may be one having a structure selected from the following Formulas 4-A to 4-R:

| Formula | Structure | Formula | Structure |
|---|---|---|---|
| 4-A | 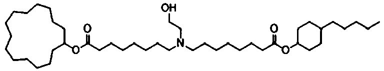 | 4-J | 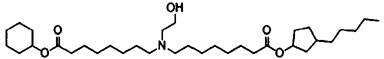 |
| 4-B | 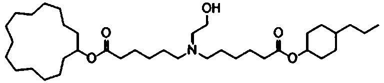 | 4-K | 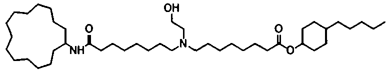 |
| 4-C | 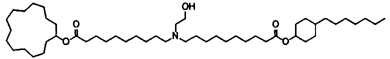 | 4-L | 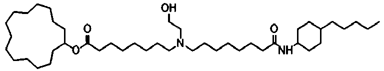 |
| 4-D | 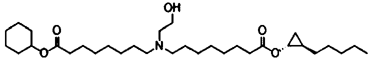 | 4-M | 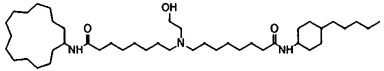 |
| 4-E | 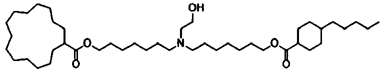 | 4-N | 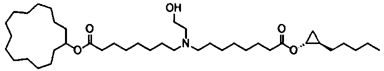 |
| 4-F | 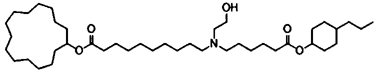 | 4-O | 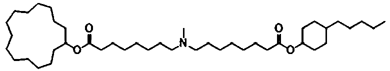 |
| 4-G | 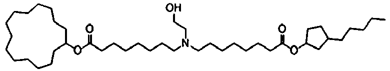 | 4-P | 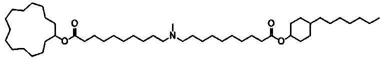 |
| 4-H | 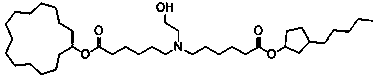 | 4-Q | 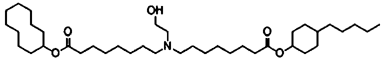 |
| 4-I | 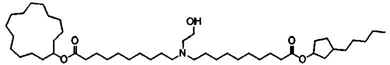 | 4-R | 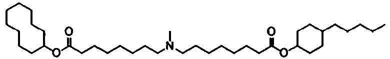 |

In another embodiment, the cationic lipid may be a lipid having a structure represented by the following Formula 5: 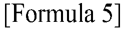 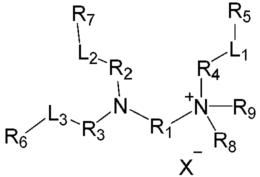
wherein, in the above Formula 5,
R₁ is a substituted or unsubstituted alkylene group, alkenylene group or alkynylene group,
each of R₂, R₃ and R₄ is independently a substituted or unsubstituted alkylene group, alkenylene group or alkynylene group,
each of R₅, R₆ and R₇ is independently a substituted or unsubstituted, saturated or unsaturated monovalent hydrocarbon group,
each of R₈ and R₉ is independently a substituted or unsubstituted alkyl group, alkenyl group, alkynyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, alkynylene, arylene, and heteroarylene, wherein L' is a direct bond, alkylene, alkenylene or alkynylene, and each R' is independently selected from the group consisting of hydrogen atom, alkyl, alkenyl and alkynyl,
n is 0 or 1, and
X⁻ is a pharmaceutically acceptable monovalent anion.

In the above Formula 5, the expression "substituted or unsubstituted" for any group means that, unless specified otherwise, the group is not substituted, or is substituted with one or more substituents selected from -OH, halogen atom, C₁₋₆ alkyl group, C₁₋₆alkoxy group, C₁₋₆halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

In the above Formula 5, the expression "hetero-" for any group (e.g., heteroaryl, heterocycloalkyl, etc.) means that, unless specified otherwise, the group has one or more (e.g., 1 to 3) heteroatoms selected from N, O and S.

In the above Formula 5, "monovalent hydrocarbon group" may be branched or unbranched, cyclic or acyclic, or aromatic.

According to an embodiment, in the above Formula 5,
R₁ may be a substituted or unsubstituted C₁₋₆ alkylene group, C₂₋₆ alkenylene group or C₂₋₆ alkynylene group,
each of R₂, R₃ and R₄ may be independently a substituted or unsubstituted C₃₋₁₂ alkylene group, C₃₋₁₂ alkenylene group or C₃₋₁₂ alkynylene group,
each of R₅, R₆ and R₇ may be independently a substituted or unsubstituted, saturated or unsaturated monovalent C₃₋₂₀ hydrocarbon group,
each of R₈ and R₉ may be independently a substituted or unsubstituted C₁₋₆ alkyl group, C₂₋₆ alkenyl group, C₂₋₆ alkynyl group or C₃₋₆ carbocyclic group, or may be independently -R₁₀-(L₄)ₙ-R₁₁,
each R₁₀ may be independently a substituted or unsubstituted C₃₋₁₂ alkylene group,
each R₁₁ may be independently a substituted or unsubstituted, saturated or unsaturated monovalent C₃₋₂₀ hydrocarbon group,
each of L₁, L₂, L₃ and L₄ may be independently selected from the group consisting of - C(O)O-, -OC(O)-, -OC(O)-L'-C(O)O-, -C(O)N(R')-, -N(R')C(O)-, -C(O)-, -C(S)-, -C(S)S-, - SC(S)-, -CH(OH)-, -P(O)(OR')O-, -S(O)₂-, -S-S-, C₂₋₆ alkenylene, C₂₋₆ alkynylene, C₆₋₂₀ arylene, and C₃₋₂₀ heteroarylene, wherein L' may be a direct bond, C₁₋₁₃ alkylene, C₂₋₁₃ alkenylene or C₂₋₁₃ alkynylene, and each R' may be independently selected from the group consisting of hydrogen atom, C₁₋₁₈ alkyl, C₂₋₁₈ alkenyl and C₂₋₁₈ alkynyl,
n is 0 or 1, and
X⁻ may be a pharmaceutically acceptable monovalent anion of inorganic acid or organic acid.

More specifically, in the above Formula 5,
R₁ may be a substituted or unsubstituted C₃₋₄ alkylene group, C₃₋₄ alkenylene group or C₃₋₄ alkynylene group,
each of R₂, R₃ and R₄ may be independently a substituted or unsubstituted C₆₋₈ alkylene group, C₆₋₈ alkenylene group or C₆₋₈ alkynylene group,
each of R₅, R₆ and R₇ may be independently a substituted or unsubstituted, saturated or unsaturated monovalent C₅₋₁₅ hydrocarbon group,
each of R₈ and R₉ may be independently a substituted or unsubstituted C₁₋₂ alkyl group, C₂₋₃ alkenyl group or C₂₋₃ alkynyl group, or may be independently -R₁₀-(L₄)ₙ-R₁₁,
each R₁₀ may be independently a substituted or unsubstituted C₆₋₈ alkylene group,
each R₁₁ may be independently a substituted or unsubstituted, saturated or unsaturated monovalent C₅₋₁₅ hydrocarbon group,
each of L₁, L₂, L₃ and L₄ may be independently selected from the group consisting of - C(O)O-, -OC(O)-, -C(O)N(R')-, -N(R')C(O)-, -P(O)(OR')O-, -S-S-, C₂₋₅ alkenylene and C₂₋₅ alkynylene, wherein each R' may be independently selected from the group consisting of hydrogen atom, C₁₋₆ alkyl, C₂₋₆ alkenyl and C₂₋₆ alkynyl,
n is 0 or 1, and
X⁻ may be halide (F⁻, Cl⁻, Br⁻, I⁻), nitrate anion (NO₃⁻), benzoate anion (C₆H₅COO⁻), methanesulfonate anion, acetate anion (CH₃COO⁻) (i.e., AcO⁻), or trihaloacetate anion (CF₃COO⁻).

Even more specifically, in the above Formula 5,
R₁ may be a substituted or unsubstituted C₃₋₄ alkylene group,
each of R₂, R₃ and R₄ may be independently a substituted or unsubstituted C₆₋₈ alkylene group,
each of R₅, R₆ and R₇ may be independently a substituted or unsubstituted, saturated or unsaturated monovalent C₅₋₁₅ hydrocarbon group,
each of R₈ and R₉ may be independently a substituted or unsubstituted C₁₋₂ alkyl group, or may be independently -R₁₀-(L₄)ₙ-R₁₁,
each R₁₀ may be independently a substituted or unsubstituted C₆₋₈ alkylene group,
each R₁₁ may be independently a substituted or unsubstituted, saturated or unsaturated monovalent C₅₋₁₅ hydrocarbon group,
each of L₁, L₂, L₃ and L₄ may be independently selected from the group consisting of - C(O)O-, -OC(O)-, -C(O)N(R')-, -N(R')C(O)-, -P(O)(OR')O-, -S-S-, and C₂₋₅ alkenylene, wherein each R' may be independently selected from the group consisting of hydrogen atom and C₁₋₆ alkyl,
n is 0 or 1, and
X⁻ may be Cl⁻, Br⁻, or acetate anion (CH₃COO⁻) (i.e., AcO⁻).

Even more specifically, the cationic lipid may be one having a structure selected from the following Formulas 5-A to 5-Q:

| Formula | Structure |
|---|---|
| 5-A | 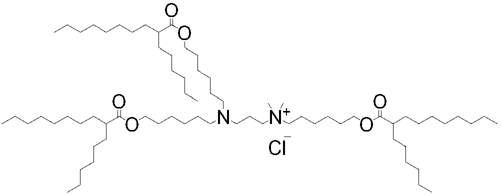 |
| 5-B | 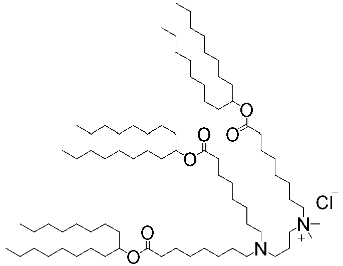 |
| 5-C | 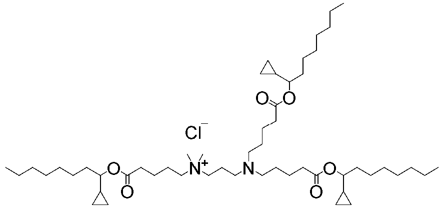 |
| 5-D | 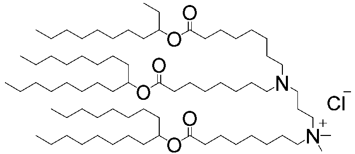 |
| 5-E | 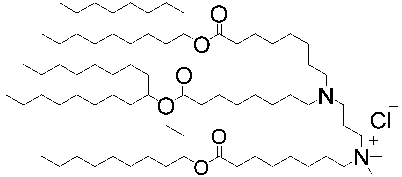 |
| 5-F | 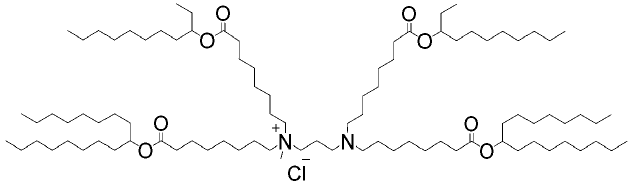 |
| 5-G | 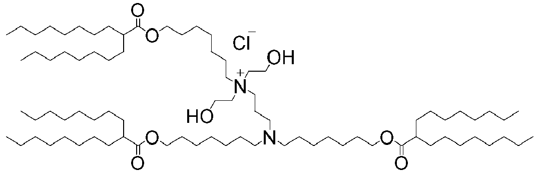 |
| 5-H | 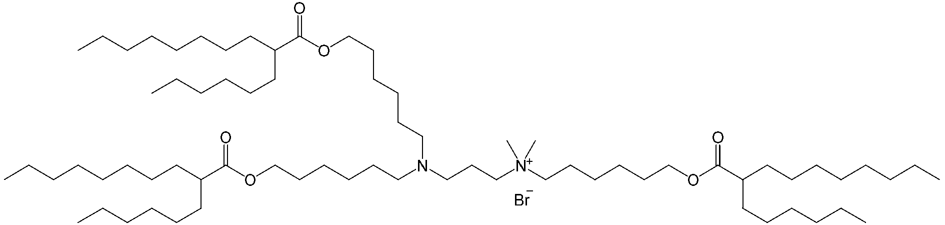 |
| 5-I | 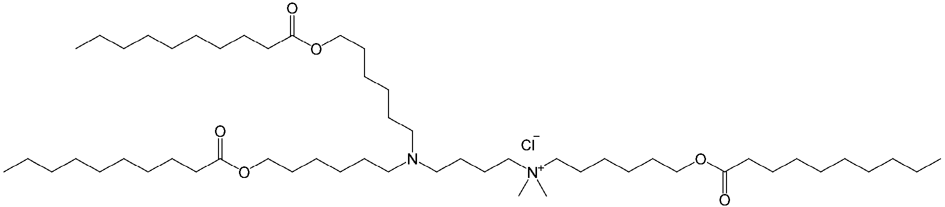 |
| 5-J | 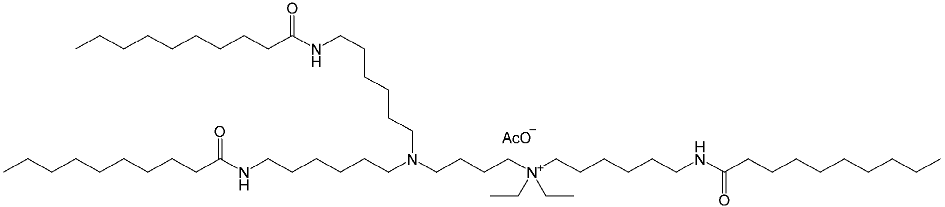 |
| 5-K | 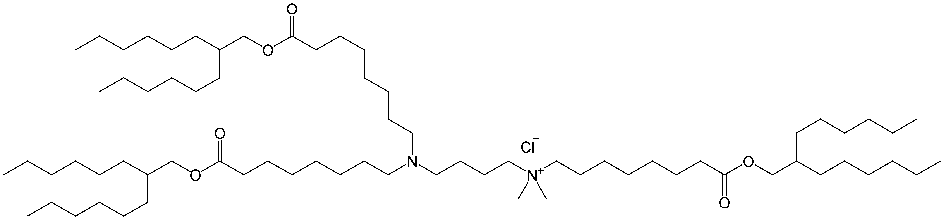 |
| 5-L | 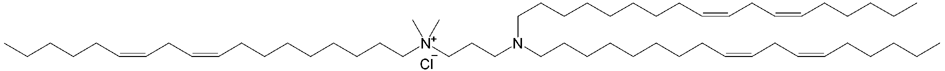 |
| 5-M | 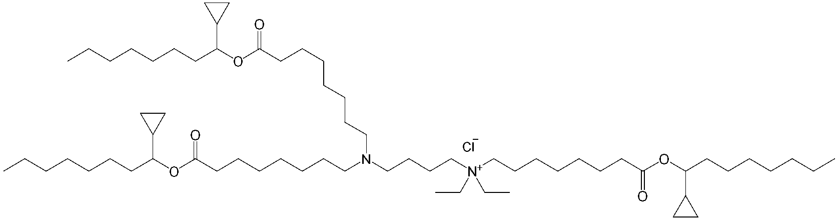 |
| 5-N | 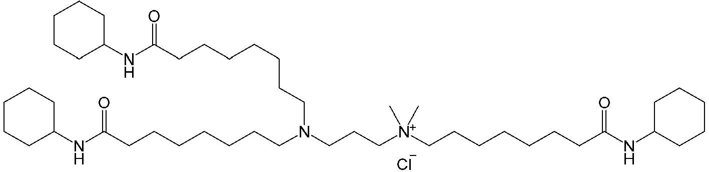 |
| 5-O | 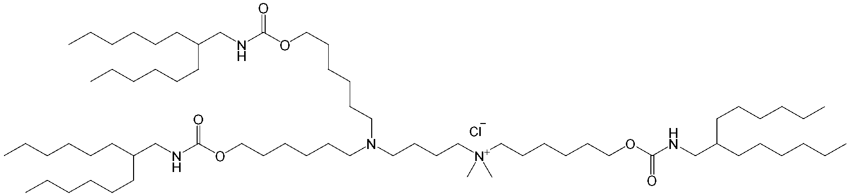 |
| 5-P | 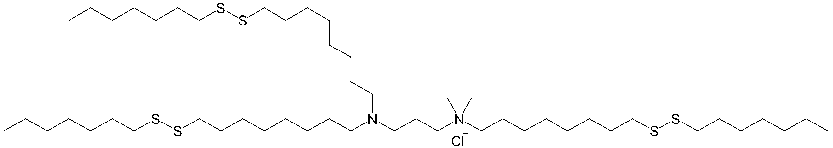 |
| 5-Q | 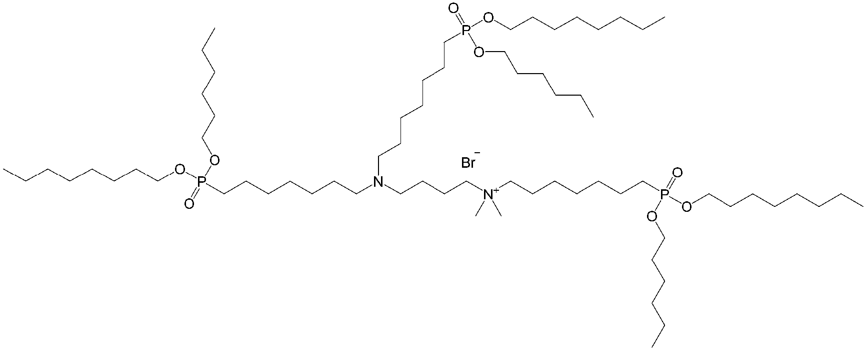 |

In another embodiment, the cationic lipid may be a lipid having a structure represented by the following Formula 6, or an ionized form thereof: 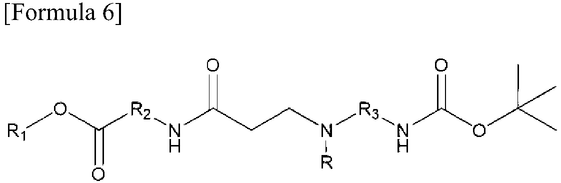
wherein, in the above Formula 6,
R₁ is a substituted or unsubstituted alkyl group, alkenyl group or alkynyl group,
R₂ is a substituted or unsubstituted alkylene group, alkenylene group or alkynylene group,
R₃ is an unsubstituted alkylene group,
R is hydrogen atom (H), or 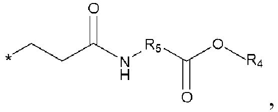 where R₄ is a substituted or unsubstituted alkyl group, alkenyl group or alkynyl group, R₅ is a substituted or unsubstituted alkylene group, alkenylene group or alkynylene group, and * indicates a point of attachment to the nitrogen atom.

In the above Formula 6, the expression "substituted or unsubstituted" for any group means that, unless specified otherwise, the group is not substituted, or is substituted with one or more substituents selected from -OH, halogen atom, C₁₋₆ alkyl group, C₁₋₆alkoxy group, C₁₋₆halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

In the above Formula 6, each of "alkyl," "alkenyl," "alkynyl," "alkylene," "alkenylene," and "alkynylene" may independently be branched or unbranched, or cyclic or acyclic.

According to an embodiment, in the above Formula 6,
each of R₁ and R₄ may be independently a substituted or unsubstituted C₁₋₃₀ alkyl group, a substituted or unsubstituted C₂₋₃₀ alkenyl group, or a substituted or unsubstituted C₂₋₃₀ alkynyl group,
each of R₂ and R₅ may be independently a substituted or unsubstituted C₁₋₁₅alkylene group, a substituted or unsubstituted C₂₋₁₅ alkenylene group, or a substituted or unsubstituted C₂₋₁₅ alkynylene group, and
R₃ may be an unsubstituted C₂₋₉ alkylene group.

More specifically, in the above Formula 6,
each of R₁ and R₄ may be independently a substituted or unsubstituted C₁₋₂₀ alkyl group, a substituted or unsubstituted C₂₋₂₀ alkenyl group, or a substituted or unsubstituted C₂₋₂₀ alkynyl group,
each of R₂ and R₅ may be independently a substituted or unsubstituted C₁₋₁₂alkylene group, a substituted or unsubstituted C₂₋₁₂ alkenylene group, or a substituted or unsubstituted C₂₋₁₂ alkynylene group, and
R₃ may be an unsubstituted C₂₋₇ alkylene group.

Still more specifically, in the above Formula 6,
each of R₁ and R₄ may be independently a substituted or unsubstituted C₅₋₂₀ alkyl group, a substituted or unsubstituted C₅₋₂₀ alkenyl group, or a substituted or unsubstituted C₅₋₂₀ alkynyl group,
each of R₂ and R₅ may be independently a substituted or unsubstituted C₃₋₁₂alkylene group, a substituted or unsubstituted C₃₋₁₂ alkenylene group, or a substituted or unsubstituted C₃₋₁₂ alkynylene group, and
R₃ may be an unsubstituted C₂₋₅ alkylene group.

Even more specifically, the cationic lipid of the above Formula 6 may be one having a structure selected from the following Formulas 6-A to 6-L:

| Formula | Structure |
|---|---|
| 6-A | 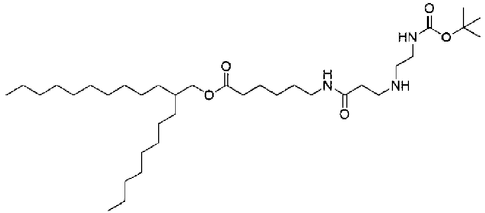 |
| 6-B | 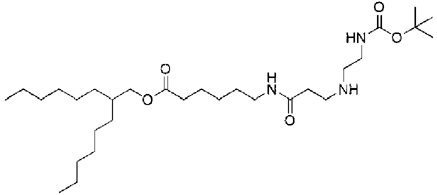 |
| 6-C | 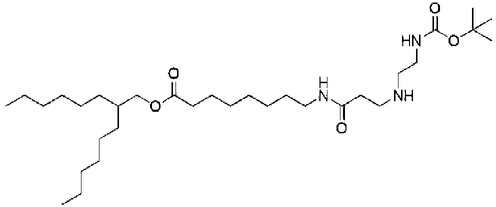 |
| 6-D | 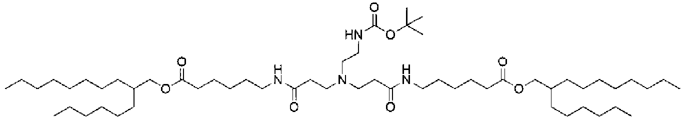 |
| 6-E | 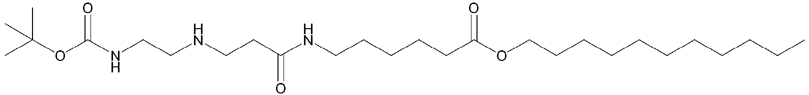 |
| 6-F | 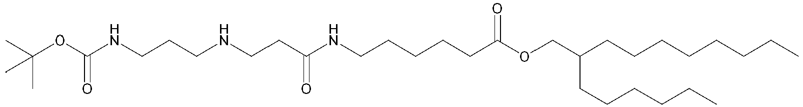 |
| 6-G | 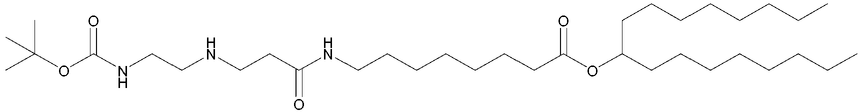 |
| 6-H | 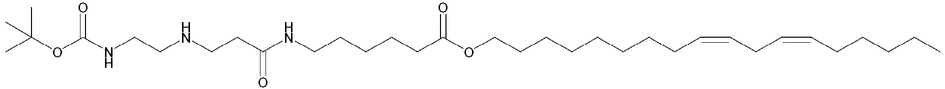 |
| 6-I | 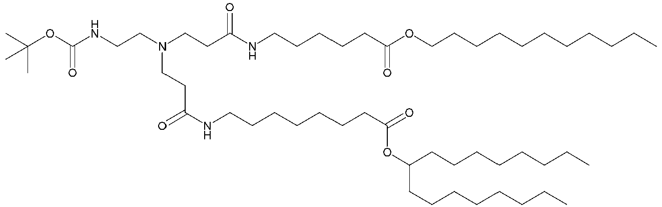 |
| 6-J | 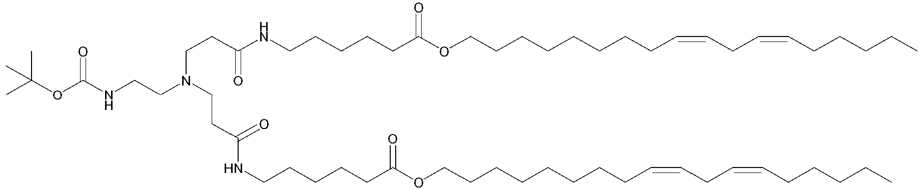 |
| 6-K | 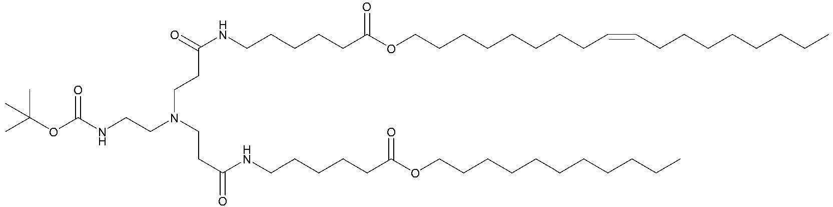 |
| 6-L | 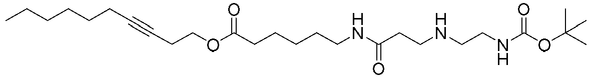 |

In another embodiment, the cationic lipid may be a lipid having a structure represented by the following Formula 7: 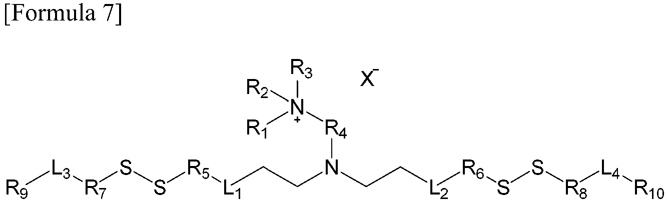
wherein, in the above Formula 7,
each of R₁, R₂ and R₃ is a substituted or unsubstituted alkyl group,
R₄ is a substituted or unsubstituted divalent hydrocarbon group,
each of R₅, R₆, R₇ and R₈ is independently a substituted or unsubstituted, saturated or unsaturated divalent hydrocarbon group,
each of R₉ and R₁₀ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O- and -S(O)₂-, wherein L' is a direct bond, alkylene, alkenylene or alkynylene, and each R' is independently selected from the group consisting of hydrogen atom, alkyl, alkenyl and alkynyl, and
X⁻ is a monovalent anion.

In the above Formula 7, the expression "substituted or unsubstituted" for any group means that, unless specified otherwise, the group is not substituted, or is substituted with one or more substituents selected from -OH, halogen atom, C₁₋₆ alkyl group, C₁₋₆alkoxy group, C₁₋₆halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

In the above Formula 7, the expression "hetero-" for any group (e.g., heteroaryl, heterocycloalkyl, etc.) means that, unless specified otherwise, the group has one or more (e.g., 1 to 3) heteroatoms selected from N, O and S.

In the above Formula 7, "monovalent hydrocarbon group" and "divalent hydrocarbon group" may be branched or unbranched, cyclic or acyclic, or aromatic.

In the above Formula 7, each of "alkyl," "alkenyl," "alkynyl," "alkylene," "alkenylene," and "alkynylene" may independently be branched or unbranched, or cyclic or acyclic.

According to an embodiment, in the above Formula 7,
each of R₁, R₂ and R₃ is a substituted or unsubstituted C₁₋₆ alkyl group,
R₄ is a substituted or unsubstituted divalent C₂₋₆ hydrocarbon group,
each of R₅, R₆, R₇ and R₈ is independently a substituted or unsubstituted, saturated or unsaturated divalent C₂₋₂₀ hydrocarbon group,
each of R₉ and R₁₀ is independently a substituted or unsubstituted, saturated or unsaturated monovalent C₂₋₂₀ hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O- and -S(O)₂-, wherein L' is a direct bond, C₁₋₁₃ alkylene, C₂₋₁₃ alkenylene or C₂₋₁₃ alkynylene, and each R' is independently selected from the group consisting of hydrogen atom, C₁₋₁₈ alkyl, C₂₋₁₈ alkenyl and C₂₋₁₈ alkynyl, and
X⁻ may be a monovalent anion of inorganic acid or organic acid.
More specifically, in the above Formula 7,
each of R₁, R₂ and R₃ is a substituted or unsubstituted C₁₋₃ alkyl group,
R₄ is a substituted or unsubstituted divalent C₂₋₄ alkylene group,
each of R₅, R₆, R₇ and R₈ is independently a substituted or unsubstituted C₂₋₁₃ alkylene group or C₂₋₁₃ alkenylene group,
each of R₉ and R₁₀ is independently a substituted or unsubstituted C₆₋₂₀ alkyl group, C₆₋₂₀ alkenyl group or C₆₋₂₀ alkynyl group,
each of L₁, L₂, L₃ and L₄ is independently selected from the group consisting of -C(O)O-, -OC(O)-, -C(O)N(R')-, -N(R')C(O)- and -P(O)(OR')O-, wherein each R' is independently selected from the group consisting of hydrogen atom and C₁₋₆ alkyl, and
X⁻ may be F⁻, Cl⁻, Br⁻, I⁻, nitrate anion, benzoate anion, methanesulfonate anion, acetate anion (CH₃COO⁻) (i.e., AcO⁻), or trihaloacetate anion (CF₃COO⁻).

Even more specifically, the cationic lipid of the above Formula 7 may be one having a structure selected from the following Formulas 7-A and 7-B:

| Formula | Structure |
|---|---|
| 7-A | 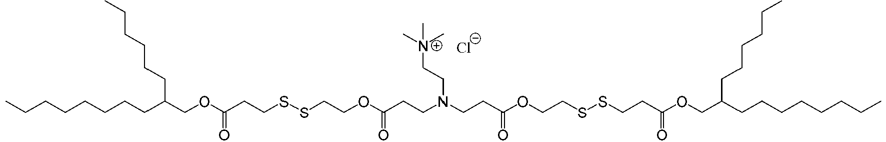 |
| 7-B | 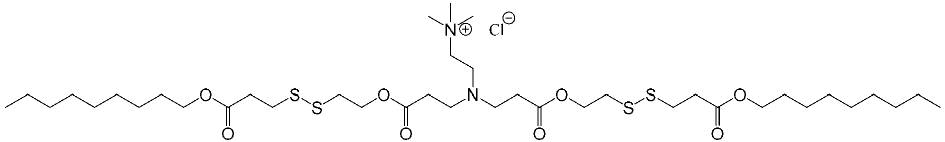 |

Meanwhile, in an embodiment, the cationic polymer may be selected from the group consisting of chitosan, glycol chitosan, protamine, polylysine, polyarginine, polyamidoamine (PAMAM), polyethylenimine, dextran, hyaluronic acid, albumin, branched polyethylenimine (PEI), polyamine and polyvinylamine (PVAm). More specifically, the cationic polymer may be one or more selected from the group consisting of polyethylenimine (PEI), polyamine and polyvinylamine (PVAm).

In an embodiment, the amount of the cationic compound in the composition for drug delivery of the present invention may be, based on the dry weight of the total composition, 5 wt% or more, 10 wt% or more, 15 wt% or more, 20 wt% or more, 25 wt% or more, 30 wt% or more, or 35 wt% or more, and it may also be 95 wt% or less, 90 wt% or less, 85 wt% or less, 80 wt% or less, 75 wt% or less, 70 wt% or less, or 65 wt% or less. If the amount of the cationic compound is too less, it may not be sufficient to form nanoparticles. To the contrary, if the amount of the cationic compound is too much, the size of the nanoparticle becomes too large, and thus the nanoparticle stability may be lowered and the rate of loss during filter sterilization may increase.

In the composition for drug delivery of the present invention, the effective ingredient is maintained in a state of being encapsulated within a nanoparticle structure formed by a polymer component comprising the amphiphilic block copolymer and the cationic compound, thereby improving stability in blood or body fluids.

In an embodiment, the particle size of the nanoparticle can be defined by Z-average value, and for example, it may be 800 nm or less, 600 nm or less, 500 nm or less, 400 nm or less, 300 nm or less, 200 nm or less, or 150 nm or less, and also may be 10 nm or more, 50 nm or more, or 100 nm or more. In an embodiment, the particle size of the nanoparticle defined by Z-average value may be, for example, 10 to 800 nm, 20 to 600 nm, 30 to 500 nm, 50 to 400 nm, or 80 to 300 nm.

In an embodiment, the relative amount of the polymer component comprising the amphiphilic block copolymer to the cationic compound may be, based on 1 part by weight of the cationic compound, 0.01 part by weight or more, 0.02 part by weight or more, 0.03 part by weight or more, 0.04 part by weight or more, or 0.05 part by weight or more, and it may also be 50 parts by weight or less, 49 parts by weight or less, 47 parts by weight or less, 45 parts by weight or less, 43 parts by weight or less, 41 parts by weight or less, 40 parts by weight or less, 39 parts by weight or less, or 37 parts by weight or less, but it is not limited thereto.

### Optional additive component

In an embodiment, in order to increase the efficiency of *in vivo* delivery of the effective ingredient, the composition for drug delivery of the present invention may further comprise fusogenic lipid.

In an embodiment, the fusogenic lipid may be one or a combination of two or more selected from the group consisting of phospholipid, PEG lipid (PEGylated lipid), cholesterol, and tocopherol.

Specifically, the phospholipid may be one or more selected from the group consisting of phosphatidylethanolamine (PE), phosphatidylcholine (PC) and phosphatidic acid. The phosphatidylethanolamine (PE), phosphatidylcholine (PC) and phosphatidic acid may be in a form combined with one or two C₁₀₋₂₄ fatty acids. The cholesterol and tocopherol include analogues, derivatives and metabolites of each of the cholesterol and tocopherol.

Specifically, the PEG lipid refers to a polyethylene glycol (PEG)-modified lipid, and is a type of PEG derivative having a lipid moiety attached thereto, such as DMG or DSPE. The PEG lipid may be used to improve the circulation time of active ingredient encapsulated in a lipid nanoparticle and to reduce non-specific uptake. The PEG lipid may be one or more selected from the group consisting of PEG-modified phosphatidylethanolamines, PEG-modified phosphatidic acids, PEG-modified ceramides, PEG-modified dialkylamines, PEG-modified diacylglycerols, and PEG-modified dialkylglycerols, and combinations thereof. For example, the PEG lipid includes 1,2-dimyristoyl-sn-glycero methoxypolyethylene glycol (PEG-DMG), 1,2-distearoyl-sn-glycero-3-phosphoethanolamine-N-[amino(polyethylene glycol)] (PEG-DSPE), PEG-distearyl glycerol (PEG-DSG), PEG-dipalmitoyl, PEG-dioleyl, PEG-distearyl, PEG-diacylglycamide (PEG-DAG), PEG-dipalmitoyl phosphatidylethanolamine (PEG-DPPE), or PEG-1,2-dimyristyloxypropyl-3-amine (PEG-c-DMA).

Specifically, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

More specifically, the fusogenic lipid may be one or a combination of two or more selected from the group consisting of dioleoyl phosphatidylethanolamine (DOPE), 1,2-dipalmitoleoyl-sn-glycero-3-phosphocholine (DPPC), distearoyl phosphatidylcholine (DSPC), 1,2-dioleoyl-sn-glycero-3-phosphocholine (DOPC), 1-octadecanoyl-sn-glycero-3-phosphocholine (18:0 Lyso PC), 1-(9Z-octadecenoyl)-sn-glycero-3-phosphocholine (18:1 Lyso PC), 1-palmitoyl-2-oleoyl-sn-glycero-3-phosphocholine, 1-stearoyl-2-oleoyl-sn-glycero-3-phosphocholine (SOPC), 1,2-dioleoyl-sn-glycero-3-phosphocholine (DOPC), 1-oleoyl-2-hydroxy-sn-glycero-3-phosphoethanolamine (18:1 Lyso PE), 1-stearoyl-2-oleoyl-sn-glycero-3-phosphoethanolamine (18:0-18:1 PE), 1-stearoyl-2-linoleoyl-sn-glycero-3-phosphoethanolamine (18:0-18:2 PE), 1,2-dilinoleoyl-sn-glycero-3-phosphoethanolamine (18:2 PE), 1-stearoyl-2-hydroxy-sn-glycero-3-phospho-(1'-rac-glycerol) (18:0 Lyso PG), 1-oleoyl-2-hydroxy-sn-glycero-3-phospho-(1'-rac-glycerol) (18:1 Lyso PG), 1,2-dipalmitoleoyl-sn-glycero-3-phosphoethanolamine (DPPE), 1,2-dioleoyl-3-trimethylammonium-propane (DOTAP), 1,2-dioleoyl-3-dimethylammonium-propane (DODAP), 1,2-dioleoyl-sn-glycero-3-phosphate (18PA), cholesterol, and tocopherol.

In an embodiment of the present invention, the fusogenic lipid may be distearoyl phosphatidylcholine, cholesterol, or a combination thereof.

In an embodiment, the amount of the fusogenic lipid may be, based on the dry weight of the total composition, 1 wt% or more, 2 wt% or more, 3 wt% or more, 4 wt% or more, or 5 wt% or more, and it may also be 40 wt% or less, 35 wt% or less, 30 wt% or less, 25 wt% or less, or 20 wt% or less.

In an embodiment, the relative amount of the fusogenic lipid to the cationic compound may be, based on 1 part by weight of the cationic compound, 0.05 part by weight or more, 0.06 part by weight or more, 0.07 part by weight or more, 0.08 part by weight or more, 0.09 part by weight or more, or 0.1 part by weight or more, and it may also be 6 parts by weight or less, 5.5 parts by weight or less, 5 parts by weight or less, 4.5 parts by weight or less, 4 parts by weight or less, or 3.8 parts by weight or less, but it is not limited thereto.

In an embodiment, in case of using phospholipid as the fusogenic lipid, its relative amount may be, based on 1 part by weight of the cationic compound, 0.03 part by weight or more, 0.04 part by weight or more, 0.05 part by weight or more, or 0.06 part by weight or more, and it may also be 4 parts by weight or less, 3.9 parts by weight or less, 3.7 parts by weight or less, 3.5 parts by weight or less, 3.3 parts by weight or less, 3.1 parts by weight or less, 3 parts by weight or less, 2.9 parts by weight or less, or 2.7 parts by weight or less, but it is not limited thereto.

In an embodiment, in case of using cholesterol as the fusogenic lipid, its relative amount may be, based on 1 part by weight of the cationic compound, 0.02 part by weight or more, 0.03 part by weight or more, or 0.04 part by weight or more, and it may also be 2 parts by weight or less, 1.9 parts by weight or less, 1.7 parts by weight or less, 1.5 parts by weight or less, 1.3 parts by weight or less, or 1.1 parts by weight or less, but it is not limited thereto.

Also, in an embodiment, the composition for drug delivery of the present invention may further comprise one or more additive components commonly included in a drug delivery composition (hereinafter, "optional additive component(s)").

In an embodiment, the optional additive component(s) may be, for example, one or more selected from the group consisting of pH modifiers (e.g., acidifying agents, alkalizing agents, buffering agents), tonicity modifiers, bulking agents (e.g., sugars, polyols, amino acids, polymers, proteins, etc.), wetting agents, solubilizing agents, surfactants, antioxidants, antimicrobial agents, chelating agents, complexing agents, etc., but it is not limited thereto.

In an embodiment, the pH modifier may be one or more selected from the group consisting of acetate, citrate, tartrate, histidine, glutamate, phosphate, Tris, glycine, bicarbonate, succinate, sulfate, nitrate, etc., but it is not limited thereto.

In an embodiment, the tonicity modifier may be one or more selected from the group consisting of mannitol, sorbitol, lactose, dextrose, trehalose, sodium chloride, potassium chloride, glycerol, glycerin, propylene glycol, etc., but it is not limited thereto.

In an embodiment, the bulking agent may be one or more selected from the group consisting of sugars and polyols including sucrose, trehalose, glucose, lactose, sorbitol, mannitol, glycerol, etc.; amino acids including arginine, aspartic acid, glutamic acid, lysine, proline, glycine, histidine, methionine, alanine, etc.; polymers and proteins including gelatin, polyvinylpyrrolidone (PVP), poly(lactide-co-glycolide) (PLGA), polyethylene glycol (PEG), dextran, cyclodextran, or derivatives thereof, starch derivatives, hydroxylamine sulfate (HAS), bovine serum albumin (BSA), etc.; or combinations thereof, but it is not limited thereto.

In an embodiment, the wetting agent and/or solubilizing agent may be one or more selected from the group consisting of lecithin, PEG 300, PEG 600, PEG 1000, polyoxyethylene lauryl ethers (e.g., Brij 30, Brij 35, Brij 56, Brij 76, Brij 97), polypropylene glycol (PPG) 2000, glucoside alkyl ethers, polyoxyethylene glycol octylphenol ethers, polyoxyethylene glycol alkylphenol ethers, glycerol alkyl esters, polysorbate 20, polysorbate 40, polysorbate 60, polysorbate 80, sorbitan monolaurate (Span 20), sorbitan monooleate (Span 80), sorbitan trioleate (Span 85), cocamide monoethanolamine (MEA), cocamide diethanolamine (DEA), dodecyldimethylamine oxide, poloxamer, polyvinyl pyrrolidone K25, polyvinyl alcohol, oligolactic acid, sodium dioctyl sulfosuccinate, diethylene glycol monolaurate, triethanolamine oleate, sodium oleate, potassium oleate, ethyl oleate, oleic acid, ethyl laurate, sodium lauryl sulfate, etc., but it is not limited thereto.

In an embodiment, the antioxidant may be one or more selected from the group consisting of tocopherol (vitamin E), alpha tocopherol, alpha tocopherol hydrogen succinate, ascorbic acid, ascorbyl palmitate, butylated hydroxy anisole (BHA), butylated hydroxy toluene (BHT), monothioglycerol, potassium metabisulfite, propionic acid, propyl gallate, sodium ascorbate, sodium bisulfite, sodium metabisulfite, sodium sulfite, histamine, methionine, glutathione, poly(ethylamine), etc., but it is not limited thereto.

In an embodiment, the antimicrobial agent may be one or more selected from the group consisting of benzalkonium chloride, benzethonium chloride, benzyl alcohol, bronopol, cetrimide, cetylpyridinium chloride, chlorhexidine, chlorobutanol, chlorocresol, chloroxylenol, cresol, metacresol, ethyl alcohol, glycerin, hexetidine, imidurea, phenol, phenoxyethanol, phenylethyl alcohol, phenylmercuric nitrate, propylene glycol, thimerosal, etc., but it is not limited thereto.

In an embodiment, the chelating agent may be one or more selected from the group consisting of ethylenediaminetetraacetic acid (EDTA), edetate disodium, dipotassium edetate, edetic acid, fumaric acid, malic acid, phosphoric acid, sodium edetate, tartaric acid, diethylenetriamine pentaacetic acid (DPTA), citric acid, hexaphosphate, thioglycolic acid, zinc, etc., but it is not limited thereto.

When the optional additive component is used in the composition for drug delivery of the present invention, the amount of each additive may be, for example, 0.01 wt% or more, 0.05 wt% or more, or 0.1 wt% or more, and it may also be 10 wt% or less, 5 wt% or less, or 1 wt% or less, based on the dry weight of the total composition, but it is not limited thereto.

### Composition and preparation method thereof

The composition for drug delivery according to the present invention can be administered through routes of administration such as blood vessels, muscles, mucous membranes, subcutaneous, intradermal, oral, bone, transdermal, or local tissues, and can be formulated into various oral or parenteral formulations suitable for such routes of administration. Examples of the oral formulations include various ones such as tablets, capsules, powder formulations, liquid formulations, etc., and examples of the parenteral formulations include various ones such as eye drops, injections, etc., and in an embodiment, the composition may be an injection formulation. For example, when the composition according to the present invention is freeze-dried, it can be reconstituted with distilled water for injection, 0.9% physiological saline, 5% aqueous dextrose solution, etc. to produce an injection formulation.

The present invention also provides a method for preparing a composition for drug delivery, comprising the steps of: (a) preparing a solution in which an amphiphilic block copolymer comprising a hydrophilic block and a hydrophobic block; and a cationic compound; are dissolved in a water-miscible organic solvent; and (b) to the solution prepared in step (a), adding effective ingredient selected from nucleic acid, polypeptide, virus, or combination thereof, and mixing them, wherein the hydrophobic block is a biocompatible, biodegradable polymer having a repeating unit represented by the above Formula 1.

In an embodiment, the water-miscible organic solvent in step (a) may be ethanol.

In an embodiment, step (a) can be performed in a solution under acidic condition.

In an embodiment, step (b) may comprise: (b-1) a step of preparing a buffer solution containing the effective ingredient; and (b-2) a step of adding the buffer solution of the effective ingredient prepared in step (b-1) to the solution prepared in step (a), and mixing them.

In an embodiment, the mixing ratio of the buffer solution of the effective ingredient prepared in step (b-1) to the solution prepared in step (a) may be 1:1 to 1:5 in volume ratio, and more specifically, it may be 1:2 to 1:4.

In another embodiment, step (b) may comprise: (b-1) a step of adding the effective ingredient to the solution prepared in step (a); and (b-2) a step of adding a buffer solution to the resulting mixture of step (b-1) and mixing them.

In an embodiment, the method for preparing a composition for drug delivery may further comprise a step of adding a buffer for modifying pH, water for injection, or a combination thereof to the resulting mixture of step (b).

In another embodiment, the method for preparing a composition for drug delivery may further comprise a step of removing the solvent from the resulting mixture of step (b) and then adding a freeze-drying aid thereto and freeze-drying the resulting mixture.

The freeze-drying aid is added to help the freeze-dried composition maintain a cake shape or to help the composition dissolve uniformly within a short period of time during the reconstitution process after freeze-drying, and specifically, it may be one or more selected from the group consisting of sugars, amino acids, polymers and proteins; for example, it may be one or more selected from the group consisting of lactose, mannitol, sorbitol, and sucrose. The amount of the freeze-drying aid may be 1 to 90 wt%, more specifically 10 to 60 wt%, based on the total dry weight of the freeze-dried composition.

The present invention will be explained below in more detail with reference to the following Examples. However, the Examples are only to illustrate the invention, and the scope of the present invention is not limited thereby in any manner.

### [EXAMPLES]

### Cationic Lipid Preparation Example 1

The compound of the following Formula 2-A was prepared as follows.

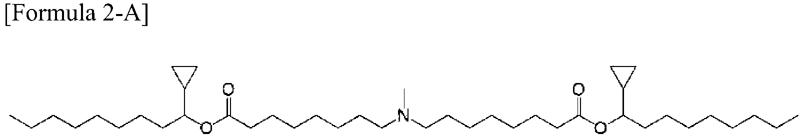

### (1) Synthesis of 1-cyclopropylnonan-1-ol

In a 2000 mL 3-neck round bottom flask (RBF), cyclopropanecarbaldehyde (35.0 g, 499 mmol, 1.00 eq) and tetrahydrofuran (THF) (700 mL) were added under a nitrogen environment and cooled to -65°C, and then octylmagnesium bromide (2 M, 375 mL, 1.50 eq) was added, and the mixture was stirred at -65°C for 2 hours. The reactor was heated to 15°C, then the reaction mixture was poured into a saturated NH₄Cl aqueous solution (500 mL), and the organic layer and aqueous layer were separated. The aqueous layer was extracted with ethyl acetate (EtOAc) (450 mL) (150 mL each, three times). The organic layers were collected, concentrated in vacuo, and purified using a silica column with petroleum ether:EtOAc = 50:1 → 0:1 to obtain 1-cyclopropylnonan-1-ol (87.5 g, 73.1%).

¹H NMR (400 MHz, CHLOROFORM-*d*): *δ* 2.93 - 2.81 (m, 1H), 1.61 (br d, 2H), 1.52 - 1.27 (m, 12H), 0.95 - 0.86 (m, 4H), 0.60 - 0.45 (m, 2H), 0.34 - 0.19 (m, 2H)

### (2) Synthesis of 1-cyclopropylnonyl 8-bromooctanoate

In a 1000 mL 3-neck RBF, 1-cyclopropylnonan-1-ol (30.0 g, 163 mmol, 1.00 eq), 8-bromooctanoic acid (72.6 g, 326 mmol, 2.00 eq), methylene chloride (DCM) (300 mL), 1-ethyl-3-(3-dimethylaminopropyl)carbodiimide (EDCI) (31.2 g, 163 mmol, 1.00 eq), and 4-dimethylaminopyridine (DMAP) (19.9 g, 163 mmol, 1.00 eq) were added, and the mixture was stirred at 25°C for 16 hours. The reaction mixture was concentrated in vacuo, and after adding silica powder thereto, it was purified using a silica column with petroleum ether:EtOAc = 10:1 → 50:1 to obtain 1-cyclopropylnonyl 8-bromooctanoate (22.8 g, 36.0%).

¹H NMR (400 MHz, CHLOROFORM-*d*): 0 4.29 (td, 1H), 3.59 - 3.31 (m, 2H), 2.32 (t, 2H), 1.94 - 1.75 (m, 2H), 1.73 - 1.60 (m, 4H), 1.49 - 1.25 (m, 18H), 1.03 - 0.93 (m, 1H), 0.90 (t, 3H), 0.61 - 0.24 (m, 4H)

### (3) Synthesis of the compound of Formula 2-A

In a 100 mL 3-neck flask, methylamine hydrochloride (173 mg, 2.57 mmol, 1.00 eq), ethanol (EtOH) (30 mL), N,N-diisopropylethylamine (DIEA) (1.66 g, 12.8 mmol, 5.00 eq), and 1-cyclopropylnonyl 8-bromooctanoate (3.00 g, 7.70 mmol, 3.00 eq) were added sequentially, and the mixture was stirred at 80°C for 72 hours. The reaction mixture was concentrated in vacuo, and after adding silica powder thereto, it was purified using a silica column with petroleum ether:EtOAc = 10:1 → 1:1 to obtain the compound of Formula 2-A (660 mg, 38.9%).

¹H NMR (400 MHz, CHLOROFORM-*d*): *δ* 4.29 (td, 2H), 2.32 (br t, 8H), 2.22 (s, 3H), 1.74 - 1.60 (m, 8H), 1.54 - 1.42 (m, 4H), 1.39 - 1.23 (m, 36H), 1.02 - 0.87 (m, 8H), 0.61 - 0.23 (m, 8H)

### Cationic Lipid Preparation Example 2

The compound of the following Formula 4-A was prepared as follows.

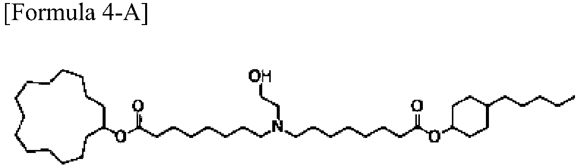

### (1) Synthesis of 4-pentylcyclohexyl 8-bromooctanoate

In a 250 mL 3-neck round bottom flask (RBF), 8-bromooctanoic acid (2.00 g, 8.96 mmol, 1.00 eq), dichloromethane (DCM) (40 mL), and dimethylformamide (DMF) (0.5 mL) were added together, and oxalyl chloride (2.28 g, 17.9 mmol, 2.00 eq) was added thereto at 0 °C under a nitrogen environment. The resulting mixture was stirred at 25 °C for 4 hours under a nitrogen environment, and then 4-pentylcyclohexan-1-ol (2.29 g, 13.5 mmol, 1.50 eq) and triethylamine (TEA) (1.36 g, 13.5 mmol, 1.50 eq) were added, and the mixture was stirred at 25 °C for an additional 12 hours under a nitrogen environment. The reaction mixture was concentrated in vacuo, and the concentrated residue was purified using a silica column with petroleum ether:ethyl acetate (EtOAc) = 1:0 → 50:1 to obtain 4-pentylcyclohexyl 8-bromooctanoate (2.46 g, 6.55 mmol, 73.1% yield) as a pale yellow oil.

¹H NMR (400 MHz, CHLOROFORM-*d*): *δ* 0.76 - 0.86 (m, 3 H) 0.88 - 1.94 (m, 28 H) 2.21 (dt, 2 H) 3.33 (td, 2 H) 4.52 - 4.66 (m, 1 H) 4.87 - 4.95 (m, 1 H)

### (2) Synthesis of 4-pentylcyclohexyl 8-((2-hydroxyethyl)amino)octanoate

In a 100 mL 3-neck RBF, 4-pentylcyclohexyl 8-bromooctanoate (2.46 g, 6.55 mmol, 1.00 eq), 2-aminoethan-1-ol (2.00 g, 32.8 mmol, 5.00 eq), and ethanol (EtOH) (50 mL) were added, and the mixture was stirred at 80 °C for 16 hours under a nitrogen environment. The reaction mixture was concentrated in vacuo, and the concentrated residue was purified using a silica column with petroleum ether:EtOAc = 1:0 → 50:1 to obtain 4-pentylcyclohexyl 8-((2-hydroxyethyl)amino)octanoate (2.00 g, 5.62 mmol, 85.8 % yield) as a yellow solid.

¹H NMR (400 MHz, CHLOROFORM-*d*): *δ* 0.76 - 0.86 (m, 3 H) 0.88 - 1.94 (m, 27 H) 2.32 (t, 2 H) 2.51 (t, 2 H) 2.71 (t, 2H), 3.54 (d, 2 H) 3.91 - 4.00 (m, 1 H) 4.11 - 4.31 (m, 1 H)

### (3) Synthesis of cyclopentadecyl 8-bromooctanoate

In a 250 mL 3-neck RBF, cyclopentadecanol (5.00 g, 22.1 mmol, 1.00 eq), 8-bromooctanoic acid (4.93 g, 22.1 mmol, 1 eq), sulfuric acid (H2SO4) (217 mg, 2.21 mmol, 0.10 eq), and toluene (100 mL) were added, and the mixture was stirred at 120 °C for 16 hours under a nitrogen environment. After evaporating the solvent, the residue was purified using a silica column with petroleum ether:EtOAc = 1:0 → 50:1 to obtain cyclopentadecyl 8-bromooctanoate (2.60 g, 6.03 mmol, 27.3% yield) as a colorless oil.

¹H NMR (400 MHz, CHLOROFORM-*d*): δ 4.89 (quin, 1H), 3.41 (dt, 2H), 2.28 (t, 2H), 1.85 (quin, 2H), 1.73 - 1.16 (m, 36H)

### (4) Synthesis of the compound of Formula 4-A

In a 100 mL 3-neck RBF, cyclopentadecyl 8-bromooctanoate (1.60 g, 3.71 mmol, 1.00 eq), 4-pentylcyclohexyl 8-((2-hydroxyethyl)amino)octanoate (1.32 g, 3.71 mmol, 1.00 eq), N,N-diisopropylethylamine (DIEA) (527 mg, 4.08 mmol, 1.10 eq), and EtOH (30 mL) were added, and the mixture was stirred at 80 °C for 48 hours. Subsequently, after evaporating the solvent, the residue was purified using a silica column with petroleum ether:EtOAc = 10:1 → 1:1, and purified again by prep-HPLC (Folic Acid condition) and washed with NaHCO₃ aqueous solution (300 mL), and then the organic layer was concentrated and extracted with DCM (200 mL x 2). The organic layer was dried over anhydrous Na₂SO₄ and filtered, and the filtrate was concentrated to obtain the compound of Formula 4-A (0.240 g, 340 µmol, 9.16% yield) as a yellow oil.

¹H NMR (400 MHz, CHLOROFORM-*d*): *δ* 4.91 (br s, 1H), 4.82 (quin, 1H), 3.46 (br t, 2H), 2.51 (br d, 2H), 2.37 (br t, 4H), 2.21 (td, 4H), 1.58 - 1.42 (m, 14H), 1.33 - 1.17 (m, 52H), 0.83 - 0.80 (m, 3H).

### Amphiphilic Block Copolymer (mPEG-PNL) Preparation Example 1

The amphiphilic block copolymer of the following formula was prepared as follows.

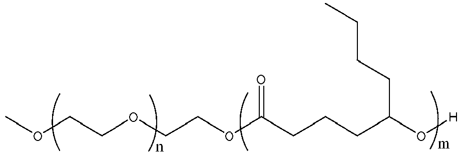

Monomethoxy polyethylene glycol (monomethoxy PEG) (5.00 g, 2.5 mmol, 1.0 eq) was charged into a 100 mL two-neck round-bottom flask (RBF) and dried at 120°C for 2 hours. δ-Nonalactone, which had been vacuum-dried at room temperature for 2 hours, and 1,5,7-triazabicyclo[4.4.0]dec-5-ene (TBD) were sequentially added to the polymerization reactor containing the monomethoxy PEG, and the reaction mixture was stirred at 50°C for 1 hour. Upon completion of the reaction, the reaction mixture was added dropwise to cold diethyl ether to induce a first precipitation. The resulting precipitate was collected by centrifugation. The precipitate was dissolved in methylene chloride (DCM), and the resulting solution was added dropwise to cold diethyl ether to induce a second precipitation. The resulting precipitate was collected by centrifugation and dissolved in DCM, followed by dropwise addition to cold hexane to induce a third precipitation. The resulting precipitate was collected by centrifugation and dissolved in DCM, followed by dropwise addition to cold hexane to induce a fourth precipitation, thereby obtaining the amphiphilic block copolymer.

¹H-NMR (400 MHz, CDCl₃) *δ* 4.88-4.87 (CH-O-CO, m), 4.22-4.20 (CH₂-O-CO, m), 3.83-3.54 (CH-OH, O-CH₂-CH₂-O, m), 3.34 (O-CH₃, s), 2.34-2.28 (O-CO-CH₂, m), 1.72-1.25 (CH₂-CH₂-CH₂-CH₃, m), 0.91-0.87 (CH₂-CH₃, m)

### Comparative Example 1

A drug delivery composition was prepared according to the composition shown in Table 1 as follows. A solution of Lipid5 (8-[(2-hydroxyethyl)[8-(nonyloxy)-8-oxooctyl]amino]-octanoic acid, 1-octylnonyl ester) (11.4 mg) in ethanol (570 µL), a solution of DSPC (distearoylphosphatidylcholine) (2.5 mg) in ethanol (250 µL), a solution of cholesterol (4.8 mg) in ethanol (480 µL), and a solution of DMG-PEG (1,2-dimyristoyl-rac-glycero-3-methoxypolyethylene glycol-2000) (1.2 mg) in ethanol (120 µL) were sequentially combined and thoroughly mixed by vortexing to prepare an ethanolic solution. An aqueous active ingredient solution was prepared by mixing 1 mg of luciferase mRNA with 20 mM sodium acetate buffer (pH 4.6). The aqueous active ingredient solution was then added to the ethanolic solution such that the volume ratio of the aqueous phase to the ethanol phase was 3:1. After mixing, the resulting mixture was centrifuged using an Amicon Ultra centrifugal filter at 4,000 rpm and concentrated to one-third of its initial volume. PBS (phosphate-buffered saline) was then added to restore the mixture to its initial volume, followed by centrifugation again at 4,000 rpm to concentrate the mixture to one-third of its initial volume. This process was repeated six times to remove ethanol and to exchange the buffer with PBS while concentrating the composition. After concentration to the desired concentration, the resulting composition was sterilized by filtration through a filter having a pore size of 0.22 µm.

**[Table 1]**

| | Luciferase mRNA | Lipid5 | DSPC | Cholesterol | DMG-PEG |
|---|---|---|---|---|---|
| Comparative Example 1 | 1mg | 11.4mg | 2.5mg | 4.8mg | 1.2mg |

### Examples 1 to 18

A drug delivery composition was prepared according to the composition shown in Table 2 as follows. The lipid compound of Formula 2-A prepared in Cationic Lipid Preparation Example 1 or the lipid compound of Formula 4-A prepared in Cationic Lipid Preparation Example 2 was dissolved in ethanol at a concentration of 20 mg/mL. DOPE (1,2-dioleoyl-sn-glycero-3-phosphoethanolamine), cholesterol, and mPEG-PNL (monomethoxypolyethylene glycol-polynonalactone) prepared in Amphiphilic Block Copolymer Preparation Example 1 were dissolved in ethanol at concentrations of 10 mg/mL, 10 mg/mL, and 25 mg/mL, respectively. For each of the above components, a volume corresponding to the amount shown in Table 2 was taken from the respective solution and combined. The resulting mixture was thoroughly mixed by vortexing to prepare an ethanolic solution. An aqueous active ingredient solution was prepared by mixing 1 mg of luciferase mRNA with 20 mM sodium acetate buffer (pH 4.6). The aqueous active ingredient solution was then added to the ethanolic solution such that the volume ratio of the aqueous phase to the ethanol phase was 3:1. After mixing, the resulting mixture was vortexed for about 5 seconds to ensure thorough mixing and then centrifuged using an Amicon Ultra centrifugal filter at 4,000 rpm to concentrate the mixture to one-third of its initial volume. PBS (phosphate-buffered saline) was then added to restore the mixture to its initial volume, followed by centrifugation again at 4,000 rpm to concentrate the mixture to one-third of its initial volume. This process was repeated six times to remove ethanol and to exchange the buffer with PBS while concentrating the composition. After concentration to the desired concentration, the resulting composition was sterilized by filtration through a filter having a pore size of 0.22 µm.

**[Table 2] (unit: mg)**

| | Luciferase mRNA | Lipid of Formula 2-A | DOPE | Cholesterol | mPEG-PNL |
|---|---|---|---|---|---|
| Example 1 | 1 | 11.7 | 5.4 | 4.2 | 1.5 |
| Example 2 | 1 | 11.7 | 5.4 | 4.2 | 5.0 |
| Example 3 | 1 | 11.7 | 8.0 | 2.8 | 5.0 |
| Example 4 | 1 | 17.5 | 10.1 | 10.5 | 9.5 |
| Example 5 | 1 | 17.5 | 15.1 | 7.8 | 9.5 |
| Example 6 | 1 | 17.5 | 20.1 | 5.2 | 9.5 |
| Example 7 | 1 | 17.5 | 4.0 | 8.4 | 7.6 |
| Example 8 | 1 | 17.5 | 8.0 | 6.3 | 7.6 |
| Example 9 | 1 | 17.5 | 12.1 | 4.2 | 7.6 |
| Example 10 | 1 | 17.5 | 16.1 | 2.1 | 7.6 |
| Example 11 | 1 | 17.5 | 3.4 | 5.2 | 6.3 |
| Example 12 | 1 | 17.5 | 6.7 | 3.5 | 6.3 |
| Example 13 | 1 | 17.5 | 10.1 | 1.7 | 6.3 |
| Example 14 | 1 | 17.5 | 8.0 | 6.3 | 15.1 |
| Example 15 | 1 | 17.5 | 8.0 | 6.3 | 22.7 |
| Example 16 | 1 | 17.5 | 12.1 | 4.2 | 15.1 |
| Example 17 | 1 | 17.5 | 12.1 | 4.2 | 22.7 |

| | Luciferase mRNA | Lipid of Formula 4-A | DOPE | Cholesterol | mPEG-PNL |
|---|---|---|---|---|---|
| Example 18 | 1 | 13.1 | 5.5 | 4.3 | 5.2 |

### (3) Evaluation of Formulation Properties

For each formulation of Comparative Example 1 and Examples 1 to 18, particle characteristics were evaluated using a particle size analyzer based on dynamic light scattering (DLS), and the results are shown in Table 3 below.

**[Table 3]**

| | Zeta-average size (nm) | PD index (PI) | Zeta-potential (mV) |
|---|---|---|---|
| Comparative Example 1 | 95.8 ± 1.2 | 0.155 ±0.155 | -4.1± 4.3 |
| Example 1 | 193.0 ± 2.04 | 0.023 ± 0.006 | -14.4 ± 0.564 |
| Example 2 | 113.1 ± 1.2 | 0.078 ±0.013 | -3.1± 2.2 |
| Example 3 | 109.4 ± 1.2 | 0.032 ± 0.022 | -6.1 ± 1.7 |
| Example 4 | 85.4 ± 0.8 | 0.092 ± 0.009 | -3.1 ± 1.4 |
| Example 5 | 86.3 ± 0.5 | 0.075 ± 0.022 | -3.0 ± 2.0 |
| Example 6 | 87.5 ± 1.4 | 0.136 ± 0.012 | -1.4 ± 4.0 |
| Example 7 | 101.5 ± 0.7 | 0.067 ± 0.028 | -6.4 ± 2.9 |
| Example 8 | 108.1 ± 2.6 | 0.026 ± 0.024 | -1 ± 4.6 |
| Example 9 | 105.1 ± 2.1 | 0.03 ± 0.019 | -6.1± 5.3 |
| Example 10 | 101.2 ± 0.5 | 0.081 ± 0.010 | 0.6 ± 4.5 |
| Example 11 | 95.6 ± 0.5 | 0.057 ± 0.048 | -9.7 ± 3.1 |
| Example 12 | 100.3 ± 1.0 | 0.090 ± 0.016 | -10.1 ± 0.9 |
| Example 13 | 95.8 ± 0.7 | 0.052 ± 0.007 | -6.7 ± 4.9 |
| Example 14 | 82.5 ± 3.0 | 0.177 ± 0.024 | -5.3 ± 2.9 |
| Example 15 | 69.4 ± 1.7 | 0.162 ± 0.012 | -6.3 ± 6.6 |
| Example 16 | 72.1 ± 0.4 | 0.130 ± 0.020 | -6.3 ± 5.6 |
| Example 17 | 59.7 ± 1.1 | 0.112 ± 0.009 | -4.6 ± 2.0 |
| Example 18 | 98.1 ± 0.4 | 0.094 ± 0.010 | 2.9 ± 1.4 |

### (4) Administration of Composition

The formulations of Comparative Example 1 and Examples 1 to 10, 13, 14, 16, and 18 were prepared at a concentration of 10 µg/mL and administered intravenously to mice at a dose corresponding to 2 µg of mRNA per mouse. Four hours after administration, luciferin dissolved in sterile water was prepared at a concentration of 15 µg/µL and administered intraperitoneally at a dose of 3 mg per 20 g mouse. Fifteen minutes after intraperitoneal administration of luciferin, protein expression in individual organs was measured using a bioluminescence imaging system, and the results are shown in Table 4 below.

As can be seen from Table 4, the drug delivery formulations according to the present invention exhibited excellent efficiency for selective delivery of the drug to the liver following intravenous administration.

**[Table 4]**

| | Avg Radiance [p/s/cm²/sr] | | Liver/Spleen (Fold) |
|---|---|---|---|
| | Liver | Spleen | |
| Comparative Example 1 | 1.32E+08 | 2.11E+07 | 6.3 |
| Example 1 | 3.16E+07 | 3.37E+06 | 9 |
| Example 2 | 2.10E+07 | 2.01E+05 | 104.5 |
| Example 3 | 2.30E+07 | 2.07E+05 | 111.1 |
| Example 4 | 1.28E+07 | 2.14E+05 | 60.0 |
| Example 5 | 2.89E+07 | 3.37E+05 | 85.6 |
| Example 6 | 2.44E+07 | 2.74E+05 | 89.1 |
| Example 7 | 1.70E+07 | 1.62E+05 | 105.3 |
| Example 8 | 1.22E+08 | 1.03E+06 | 118.45 |
| Example 9 | 1.35E+08 | 6.95E+05 | 194.35 |
| Example 10 | 1.24E+07 | 2.52E+05 | 49.0 |
| Example 13 | 2.61E+07 | 1.89E+05 | 138.5 |
| Example 14 | 6.23E+06 | 1.03E+05 | 60.3 |
| Example 16 | 1.29E+06 | 3.46E+04 | 37.1 |
| Example 18 | 2.32E+07 | 5.67E+05 | 41.0 |

## Claims

1. A composition for drug delivery comprising:
effective ingredient selected from nucleic acid, polypeptide, virus or combination thereof;
an amphiphilic block copolymer comprising a hydrophilic block and a hydrophobic block; and
a cationic compound,
wherein the hydrophobic block is a biocompatible, biodegradable polymer having a repeating unit represented by the following Formula 1: 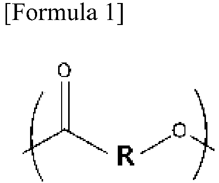
wherein, in the above Formula 1,
R represents a branched alkylene group having 3 or more carbon atoms.

2. The composition for drug delivery according to claim 1, wherein the hydrophilic block is one or more selected from the group consisting of polyalkylene glycol, polyvinyl alcohol, polyvinyl pyrrolidone, polyacrylamide, and derivatives thereof.

3. The composition for drug delivery according to claim 1, wherein R in Formula 1 represents a branched alkylene group having 3 to 20 carbon atoms.

4. The composition for drug delivery according to claim 1, wherein the hydrophobic block is a biocompatible, biodegradable polymer having repeating units of a structure selected from the following:

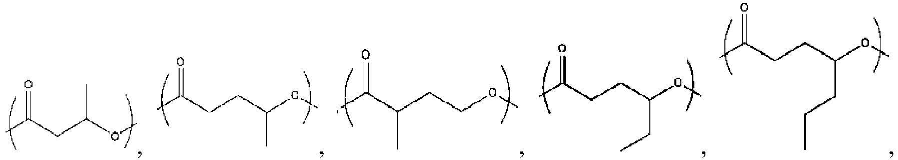

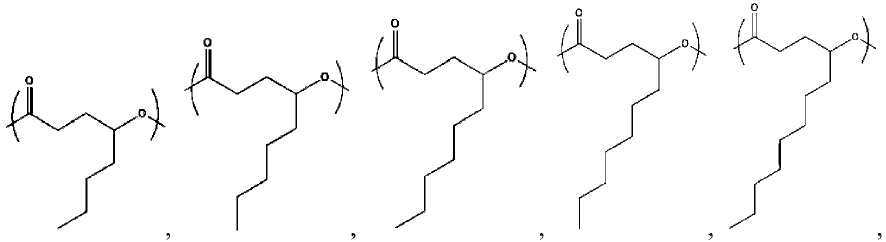

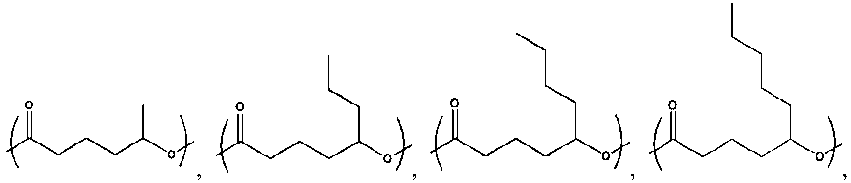

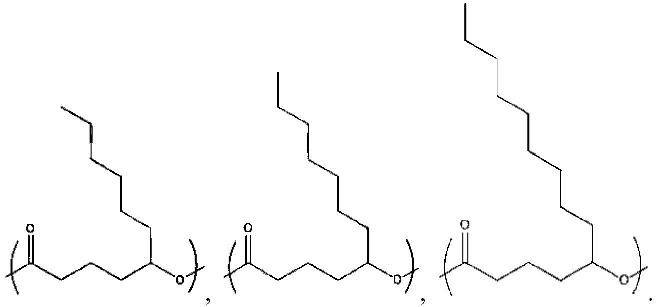

5. The composition for drug delivery according to claim 1, wherein the repeating unit represented by Formula 1 is obtained by ring-opening polymerization of a lactone compound.

6. The composition for drug delivery according to claim 1, wherein the cationic compound is a cationic lipid, a cationic polymer, or a combination thereof.

7. The composition for drug delivery according to claim 1, wherein the effective ingredient is mRNA.

8. The composition for drug delivery according to any one of claims 1 to 7, wherein the composition further comprises fusogenic lipid.

9. The composition for drug delivery according to claim 8, wherein the fusogenic lipid is one or a combination of two or more selected from the group consisting of phospholipid, PEG lipid, cholesterol, and tocopherol.

10. A method for preparing a composition for drug delivery, comprising the steps of:
(a) preparing a solution in which an amphiphilic block copolymer comprising a hydrophilic block and a hydrophobic block; and a cationic compound; are dissolved in a water-miscible organic solvent; and
(b) to the solution prepared in step (a), adding effective ingredient selected from nucleic acid, polypeptide, virus, or combination thereof, and mixing them,
wherein the hydrophobic block is a biocompatible, biodegradable polymer having a repeating unit represented by the following Formula 1: 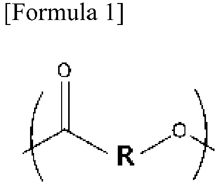
wherein, in the above Formula 1,
R represents a branched alkylene group having 3 or more carbon atoms.

11. The method for preparing a composition for drug delivery according to claim 10, wherein the water-miscible organic solvent in step (a) is ethanol.

12. The method for preparing a composition for drug delivery according to claim 10, wherein step (b) comprises:
(b-1) a step of preparing a buffer solution containing the effective ingredient; and
(b-2) a step of adding the buffer solution of the effective ingredient prepared in step (b-1) to the solution prepared in step (a), and mixing them.

13. The method for preparing a composition for drug delivery according to claim 10, wherein step (b) comprises:
(b-1) a step of adding the effective ingredient to the solution prepared in step (a); and
(b-2) a step of adding a buffer solution to the resulting mixture of step (b-1) and mixing them.
